# EUROPEAN PATENT APPLICATION

(11) **EP 4 545 551 A1**
(43) Date of publication of application: **30.04.2025**
(21) Application number: 23826351.1
(22) Date of filing: 19.06.2023
(51) Int. Cl.: C07K 7/08, A61K 38/10, A61P 35/00

(54) **PEPTIDE INHIBITOR AND USE THEREOF**

(30) Priority: 23.06.2022 CN 202210727988
(71) Applicant: Chengdu Brilliant Inspiration Biotherapeutics Co., Ltd, Chengdu, Sichuan 610041 (CN)
(72) Inventor: LI, Xiaomei, Chengdu, Sichuan 610041 (CN); DING, Yi, Chengdu, Sichuan 610041 (CN); JIANG, Qiu, Chengdu, Sichuan 610041 (CN); CHEN, Rui, Chengdu, Sichuan 610041 (CN)
(74) Representative: Vossius & Partner Patentanwälte Rechtsanwälte mbB
(86) International application number: PCT/CN2023/101094
(87) International publication number: WO 2023/246703

(57) **Abstract**

The present invention relates to a peptide inhibitor for inhibiting activation of TGF-β1 with the participation of TSP-1, or the pharmaceutically acceptable salt, solvate or prodrug thereof, and a pharmaceutical composition comprising the peptide or the pharmaceutically acceptable salt, solvate or prodrug thereof, and a method for treating or preventing TGF-β1 related diseases, in particular fibrosis and solid tumors, by using the peptide inhibitor or the pharmaceutically acceptable salt, solvate or prodrug thereof and the pharmaceutical composition.

## Description

### Technical Field

The present invention relates to a peptide inhibitor for inhibiting activation of TGF-β1 with the participation of TSP-1, or a pharmaceutically acceptable salt, solvate or prodrug thereof, and a pharmaceutical composition comprising the peptide or the pharmaceutically acceptable salt, solvate or prodrug thereof, and a method for treating or preventing TGF-β1 related diseases, in particular fibrosis and solid tumors, by using the peptide inhibitor or the pharmaceutically acceptable salt, solvate or prodrug thereof and the pharmaceutical composition.

### Background Art

Fibrosis is a pathological condition in which the fibrous connective tissue proliferates abnormally during the injury-repair process and forms tissue scar, which is characterized by excessive deposition of Extracellular Matrix (ECM). Fibrosis leads to irreversible decline in tissue and organ function, and when severe, affects the quality of life of patients and even endangers their lives. In addition, the formation of fibrosis contributes to tumorigenesis and distal metastasis, and promotes the formation of an immunosuppressive tumor microenvironment, leading to immunotherapy resistance or immune non-response.

Transforming growth factor β (TGFβ) is an important core regulatory molecule in the mechanisms of fibrosis and development and progress of tumors. TGF-β and its signaling pathway have been proposed as therapeutic targets for the treatment of fibrotic diseases and tumors. However, TGF-β, as a multifunctional cytokine, is also involved in a variety of normal physiological processes, which results in a high toxicity of drugs directly targeting TGF-β and its signaling pathway. Currently, a number of such drug development programs have been slowed or even terminated due to severe drug-related adverse responses after entering the clinical stage.

TGF-β is synthesized and secreted as an inactive precursor structure, and must undergo a series of activation events to become an active fragment before it can bind to its receptor and mediate downstream transduction pathway. The activation events of TGF-β are accomplished by different protein molecules in different environments and under different conditions, therefore, targeting a specific protein involved in the activation in disease conditions has become the latest research direction to crack the difficulty of TGF-β drug development.

Thrombospondin-1 (TSP-1) is a protein that has been clinically found to be actively expressed only in fibrosis and tumor-related indications. TSP-1 has been shown to participate in TGF-β activation events during chronic inflammation and fibrosis, and is the major activator of TGF-β1 in vivo (Cell, Vol. 93, 1159-1170, June 26, 1998, Copyright ©1998 by Cell Press). Considering the clinical applications, peptide drugs targeting TGF-β activation may offer a safer strategy than other therapeutic anti-TGF-β1 molecules due to the fact that they act at the site where TGF-β1 is activated and are easily catabolized and have low antigenicity. Therefore, the present invention intends to design polypeptide components targeting a domain of TSP-1 involved in TGF-β activation, in order to provide new peptide drugs for ameliorating fibrosis and cancer.

### Summary of Invention

Through a deliberate study, the present inventors unexpectedly discovered a group of short peptides of 5-13 amino acids comprising amino acid motif (DXED), which are not only effective in inhibiting the interaction of TSP-1 receptor with TSP-1/L-TGF-β1 complex, but also effective in blocking TSP-1-dependent L-TGF-β1 activation and the subsequent TGF-β signaling. Based on this, the present inventors have established the inhibitory peptides of the present invention (hereinafter also referred to as "TGF-β1 activation inhibiting peptide"). As shown in Examples, the inhibitory peptides of the present invention in both cyclic and linear forms exhibit inhibitory activity on TGF-β1 activation; and show stronger inhibition of TGF-β1 activation compared to a similar peptide known in the art (SEQ ID NO: 50), where the peptide length is shortened to achieve greater cost-effectiveness. In some preferred embodiments, the inhibitory peptides of the present invention also exhibit better solubility, stability and/or bioavailability compared to the known analogous peptide.

In addition, the inhibitory peptide of the present invention also exhibits favorable drug safety advantages. Studies on TSP-1 have shown that it comprises multiple functional domains, which bind independently of each other to different cell surface receptors, and are involved in a variety of physiological processes, such as hemostasis, cell adhesion, cell migration, apoptosis, and anti-angiogenesis. The inhibitory peptide of the present invention, due to its small molecular weight, high activity, and the design against the specific domain on TSP-1 involved in the activation of TGF-β1, can effectively reduce the interference with the other functional domains of TSP-1 and their related physiological functions, and safeguard the specificity and safety of the drug action.

Accordingly, in a first aspect, the present invention provides an TGF-β1 activation inhibiting peptide, or a pharmaceutically acceptable salt, solvate or prodrug thereof. The TGF-β1 activation inhibiting peptide according to the present invention is capable of specifically blocking TSP-1-involved TGF-β1 activation and has the amino acid sequence of the following general formula (I):

R¹-X¹-X²- X³-X⁴- X⁵- X⁶-X⁷-R² (I),

wherein,
R¹ is acetyl or absent;
R² is amino or absent;
X¹ is absent or selected from T and S;
X² is selected from naturally occurring amino acids or non-naturally occurring amino acids, preferably selected from polar side-chain amino acids, such as uncharged polar side-chain amino acids S, C, G, N, Q, T, Y, or negatively charged polar side-chain amino acids D and E, and more preferably selected from amino acids N, C, Q, S, T, E and D; and more preferably selected from amino acids Q, C, N, E and S;
X³ is D;
X⁴ is selected from small side-chain amino acids, such as A, C, G, P, S, T and V, more preferably selected from amino acids A, P and S, more preferably selected from amino acids A and P;
X⁵ is E;
X⁶ is D;
X⁷ is either absent or a sequence of 1, 2, 3, 4, 5, 6, or 7 amino acids, selected sequentially from the amino acid sequence of Z¹Z²Z³SZ⁴LQ, starting from the amino terminal to the carboxyl terminal;
   wherein Z¹ is an amino acid of N, Q, P, A, L, V, M, or I, preferably N, P, or L, most preferably N or P;
   Z² is an amino acid of T, S, V, C, A, K, or R, preferably T, C, or K, more preferably, if Z² is an amino acid of C or K, then Z² forms a covalent linkage with a side chain of the amino acid X² or X³,
   Z³ is an amino acid of A, V, L, or I, preferably A or V,
   Z⁴ is an amino acid of F, Y, H, or W, preferably F or H;

Optionally, if X⁴ is A, then 0 to 1 small side-chain amino acid, such as P or G, preferably P, is inserted between X³ and X⁴;

Optionally, if X⁷ consists of 1 to 5 amino acids, then the peptide has 0 to 2 additional amino acid residues added after X⁷, e.g., 1 to 2 amino acid residues selected from amino acid residues of Q, E, D and N added, or 1 amino acid residue selected from C or K added.

In some embodiments, in the peptide of the present invention,
X¹ is absent or selected from T and S;
X² is selected from amino acids Q, N, C, S, T, E and D;
X³ is D;
X⁴ is selected from A, C, G, P, S, T and V;
X⁵ is E;
X⁶ is D;
X⁷ is either absent or a sequence of 1, 2, 3, 4, 5, 6, or 7 amino acids, selected sequentially from the amino acid sequence of Z¹Z²Z³SZ⁴LQ, starting from the amino terminal to the carboxyl terminal, wherein,
   Z¹ is N, P, or L;
   Z² is T, C or K;
   Z³ is A or V;
   Z⁴ is F, Y, H, or W.

In some embodiments, in the peptide of the present invention,
X¹ is selected from T and S;
X² is selected from Q and N;
X³ is D;
X⁴ is selected from A and P;
X⁵ is E;
X⁶ is D.

In some embodiments, X⁷ is absent.

In some other embodiments, X⁷ consists of Z¹, Z¹Z², Z¹Z²Z³, Z¹Z²Z³S, Z¹Z²Z³SZ⁴, Z¹Z²Z³SZ⁴L, or Z¹Z²Z³SZ⁴LQ, and preferably, X⁷ consists of Z¹Z²Z³, Z¹Z²Z³S or Z¹Z²Z³SZ⁴,
wherein, preferably
Z¹ is N, P or L, preferably N or P;
Z² is T, K or C;
Z³ is A or V, preferably A;
Z⁴ is F, Y, H, or W, preferably F or H.

The peptide of the present invention may be a linear or cyclic peptide.

In a second aspect, the present invention also provides a therapeutic and/or preventive use of the inhibitory peptides of the present invention, or pharmaceutically acceptable salts, solvates, or prodrugs thereof, in diseases caused by or related to TSP-1-involved TGF-β1 activation (i.e., "TGF-β1 related diseases"). The present invention also provides uses of the inhibitory peptides of the present invention, or pharmaceutically acceptable salts, solvates, or prodrugs thereof, in the manufacture of a medicament for the prevention or treatment of fibrosis disorders. The fibrosis disorders are characterized by TSP-1-involved TGF-β1 activation and exhibit excessive deposition of Extracellular Matrix (ECM), but are not limited to the sites of diseases or to conventional disease classifications. The present invention also provides a use of the inhibitory peptides of the present invention in the manufacture of a medicament for the prevention or treatment of cancers associated with TGF-β1 activation.

### Brief Description of Figures

Figure 1 shows HE-stained pathological sections from a mouse fibrotic disease model. Inflammation severity assessment by HE staining shows the inhibitory effects of the polypeptides of the present invention on the inflammatory infiltration in the fibrosis pathological sites.
Figure 2 shows Masson's stained pathological sections from a mouse fibrotic disease model. Fibrosis severity assessment by MASSON'S trichrome staining shows the inhibitory effects of the polypeptides of the present invention on fibrosis deposition in the fibrosis pathological sites.
Figure 3 shows Masson's stained pathological sections from a rat fibrotic disease model, demonstrating the inhibitory effects of the polypeptides of the present invention on fibrosis deposition at the fibrosis pathological sites.

### Detailed Description

### Definition

In order that the present invention may be more readily understood, certain scientific and technical terms are specifically defined below. Unless otherwise expressly defined elsewhere herein, scientific and technical terms used herein have the meanings commonly understood by a person of ordinary skill in the art to which the present invention belongs. Amino acid residue abbreviations are standard 3-letter and/or 1-letter codes used in the art to refer to the 20 commonly used amino acids. Unless otherwise indicated, amino acid sequences are written from left to right in the amino to carboxyl direction.

The singular form used herein, including the claims, includes its corresponding plural form, unless the context clearly indicates otherwise.

The term "about" refers to a value that is within an acceptable margin of error of a particular value as determined by a person of ordinary skill in the art, the margin of error being dependent on the limitations of the measurement means, i.e., of the measurement system, used in measuring or determining the value. For example, "about" may mean within 1 or more than 1 standard deviation according to practice in the art. Alternatively, "about" may refer to a range of up to 5%,10% or 20% (i.e., ±5%, ±10% or ±20%).

The term "and/or", when used in connection with two or more options, is to be understood as meaning any one of the options or any two or more of the options.

As used herein, the terms "comprising" or "including" are intended to include the elements, integers or steps described, but not to exclude any other elements, integers or steps. When used herein, the terms "comprising" or "including" also encompass "consisting of" the elements, integers or steps referred to, unless otherwise indicated. For example, when referring to "comprising" a specific sequence, it is also intended to cover a peptide or polypeptide consisting of the specific sequence.

As used herein, the terms "peptide" and "polypeptide" are used interchangeably to refer to an amino acid sequence having 2 to 100 amino acids in length, wherein the amino acids are linked by peptide bonds. The amino acids can be naturally occurring and non-naturally occurring.

As used herein, the term "conservative amino acid substitution" or "conservative amino acid replacement" means an amino acid substitution that does not adversely affect or alter the biological function of a polypeptide comprising an amino acid sequence. Typically, a conservative amino acid substitution refers to the substitution of one amino acid for another amino acid having similar chemical properties (e.g., charge or hydrophobicity). The conservative substitution table for functionally similar amino acids is well known in the art. In the present invention, a conservative substitution residue may be derived from the following conservative substitution table, in particular is a preferable conservative amino acid substitution residue in the table below.

| Original residue | Exemplary substitution | Preferable conservative amino acid substitution |
|---|---|---|
| Ala (A) | Val; Leu; Ile | Val |
| Arg (R) | Lys; Gln; Asn | Lys |
| Asn (N) | Gln; His; Asp; Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; nor-Leu | Leu |
| Leu (L) | nor-Leu; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; nor-Leu | Leu |

### Detailed Description

In the study, the present inventors surprisingly found that a short peptide having a negatively charged amino acid-rich motif (DXED) was able to effectively trigger an inhibitory effect on TSP-1-dependent TGF-β1 activation. Through analyses such as structure-activity relationships and amino acid substitutions, the present inventors further identified essential amino acids and possible substitution sites in the peptide sequence; and carried out structural optimization through peptide cyclization and/or backbone modification, thereby establishing a series of novel inhibitory peptides against TGF-β1 activation with improved bioactivity and/or physicochemical properties.

### Inhibitory peptide of the invention

In one aspect, the present invention thus provides an TGF-β1 activation inhibiting peptide or a pharmaceutically acceptable salt, solvate or prodrug thereof. The peptide of the present invention comprises a core motif rich in negative charges (DXED) and is 5-13 amino acids long. Preferably, the peptide of the present invention consists of 7-11 amino acids. In some embodiments, the peptide of the present invention is a linear or cyclic pentapeptide or hexapeptide, such as a linear or head-to-tail cyclized hexapeptide. In some embodiments, the peptide of the present invention is a linear or cyclic heptapeptide, such as a head-to-tail cyclized heptapeptide. In some other embodiments, the peptide of the present invention is a linear or cyclic octapeptide, in particular an octapeptide which is cyclized via side chains, preferably, by a disulfide bond. In yet other embodiments, the peptide of the present invention is a linear or cyclic nonapeptide, in particular a linear nonapeptide. In yet other embodiments, the peptide of the present invention is a linear or cyclic decapeptide, e.g., a head-to-tail cyclized decapeptide. In yet other embodiments, the peptide of the present invention is a linear or cyclic undecapeptide, dodecapeptide or tridecapeptide, preferably a linear undecapeptide.

In some embodiments, the peptide of the present invention, when compared to the sequence of TQDAEDNTVSFLQ (SEQ ID NO: 51) for maximum alignment, has 0 to 5 amino acid differences, e.g., 0 to 2 amino acid differences, between the full-length peptide of the present invention and the corresponding aligned portion of SEQ ID NO: 51. The amino acid differences include amino acid substitutions, additions and deletions. For example, the peptide of the invention may be identical to the sequence SEQ ID NO: 51 in the corresponding portion, or has 1 or 2 amino acid substitutions, or has 1 to 2 amino acid additions at the N-terminal or C-terminal, and in particular at the C-terminal. The amino acids used for substitution may be naturally occurring or non-naturally occurring amino acids. In some embodiments, the peptide of the present invention comprises a proline substitution, preferably located at the second residue position of the core sequence DXED; or at the first residue position immediately adjacent to the C-terminal of the core sequence. In some embodiments, the total net charge of the peptide of the present invention is negative, e.g., -1, -2, -3 or -4, preferably -2 or -3.

In some embodiments, the peptide comprises a core amino acid sequence selected from: DAED or DPED. In other embodiments, the peptide further comprises an N or P residue located at the C-terminal of the core sequence, whereby the peptide comprises an amino acid sequence selected from: DAEDN; DPEDN; or DAEDP.

In some embodiments, the peptide of the present invention is a cyclic peptide. In some embodiments, the peptide of the present invention is cyclized by covalent linkage of the first and last amino acids. In some other embodiments, the peptide of the present invention is cyclized by a lactam bond or a disulfide bond formed between the side chains of two amino acid. To this end, a suitable amino acid or analog with an appropriate side chain group may be placed at a suitable position in the peptide of the present invention to contribute to the formation of the molecular lactam bond or disulfide bond in the peptide. In some embodiments, a lactam is formed by coupling a side chain amino functional group of an amino acid residue located at the C-terminal of the core motif (DXED) of the peptide of the present invention, to a carboxylic acid group of the amino acid at the N-terminal of the core motif (DXED). In some embodiments, a disulfide bond is formed by oxidatively coupling a cysteine located at the C-terminal of the core motif (DXED) to a cysteine located at the N-terminal of the core motif (DXED). In some embodiments of lactam cyclic peptides, a lactam bond is formed between a side chain carboxyl group of the first aspartic acid (D) of the core motif, or of an aspartic amino acid (D) or glutamic acid (E) located at the N-terminal of the core motif, and a side chain amino group of a lysine (K) located at the C-terminal of the core motif, and the two amino acids for cyclization are no less than 4 amino acids apart, e.g., 4, 5, or 6 amino acids apart, and preferably, the lysine residue for cyclization is the last residue at the C-terminal of the peptide. In some embodiments of disulfide-cyclized peptides, a disulfide bond is formed between a cysteine, which is the first amino acid immediately adjacent to N-terminal of the core motif (DXED), and a cysteine located at the C-terminal of the core motif (DXED), preferably the two amino acids for cyclization are no less than 5 amino acids apart, preferably 5 or 6 amino acids apart, and further preferably, the C-terminal cysteine for cyclization is the last residue at the C-terminal of the peptide. In some embodiments, the peptide of the invention is preferably a disulfide-cyclized peptide and preferably consists of 8 amino acids, more preferably, the N-terminal amino group of the cyclic peptide is acetylated and/or the C-terminal carboxyl group is amidated.

The peptide of the present invention may comprise a chemical modification, e.g., N-terminal acetylation, C-terminal amidation, PEG modification, lipid modification, D-type amino acid substitution, or non-naturally occurring amino acid substitution. In some embodiments, the peptide of the present invention is modified by covalent linkage to a molecule that allows the peptide to maintain its ability to inhibit TGF-β1 activation, including, for example, glycosylation, acetylation, PEGylation, phosphorylation, amidation, or derivatization utilizing a known protecting/blocking group. In an embodiment, the modification is N-terminal acylation (especially acetylation). In an embodiment, the modification is C-terminal amidation. Preferably, the peptide of the invention is an N-terminally acylated (in particular acetylated) linear peptide or side-chain cyclized peptide, and further preferably the peptide also has a C-terminal amidation. In some embodiments, the peptide may be linked to a biomolecule or to a material for binding, labeling or identification.

The TGF-β1 activation inhibiting peptide according to the present invention is capable of inhibiting TSP-1-dependent TGF-β1 activation and preferably has at least one of the following properties:
- Decreasing the amount of active TGF-β1 in fibrotic tissue;
- Inhibiting TGF-β1-mediated downstream signaling;
- Reducing collagen deposition in fibrotic tissue;
- Inhibiting inflammatory and/or fibrotic lesions mediated by TGF-β1;
- Inhibiting extracellular matrix-related gene expressions stimulated by TGF-β1;
- Inhibiting tumor cell migration;
- Preventing or treating TGF-β1 related diseases, in particular fibrosis or cancer. A person skilled in the art can determine the above properties of the peptides of the invention according to methods known in the art or described in Examples.

In some embodiments, the peptide of the present invention comprises or consists of an amino acid sequence corresponding to one of SEQ ID NOs:1-49. In some embodiments, the peptide of the present invention comprises or consists of one of SEQ ID NOs:1-49. In some embodiments, the peptide of the present invention comprises or consists of an amino acid sequence corresponding to one of SEQ ID NOs: 6, 14-15, 22-26, 33, 36, 40, 43-46, and 48-49. In some embodiments, the peptide of the present invention comprises or consists of one of SEQ ID NOs: 6, 14-15, 22-26, 33, 36, 40, 43-46, and 48-49. Preferably, the peptide of the invention blocks the binding of TSP-1 to its receptor in an assay as in Example 2. In some embodiments, the peptide of the present invention exhibits a blocking rate higher than 15%, 20%, 25%, 30%, 35%, 40% or more in an assay as in Example 2.

In some embodiments, the peptide of the present invention comprises or consists of an amino acid sequence corresponding to one of SEQ ID NOs: 3-6, 14, 17, 22, 24-26, 31, 33, 36, 40-47. In some embodiments, the peptide of the present invention comprises or consists of one of SEQ ID NOs: 3-6, 14, 17, 22, 24-26, 31, 33, 36, 40-47. In some embodiments, the peptide of the present invention comprises or consists of an amino acid sequence corresponding to one of SEQ ID NOs: 3, 6, 14, 22, 24-26, 33, 41-46. In some embodiments, a peptide of the invention comprises or consists of one of SEQ ID NOs: 3, 6, 14, 22, 24-26, 33, 41-46. Preferably, the peptide of the invention inhibits TSP-1 dependent TGF-β1 activation in an assay as in Example 3, and preferably reduces TGF-β1 activation by 25%, 30%, 35% or more compared to a negative control without the polypeptide addition.

In some embodiments, the peptide of the present invention comprises or consists of an amino acid sequence corresponding to one of SEQ ID NOs: 6, 22, 25-26, 33, 40, 43, 44, 46. In some embodiments, the peptide of the present invention comprises or consists of one of SEQ ID NOs: 6, 22, 25-26, 33, 40, 43, 44, 46. In some embodiments, the peptide of the present invention comprises or consists of SEQ ID NO: 6. In some embodiments, the peptide of the present invention comprises or consists of SEQ ID NO: 22. In some embodiments, the peptide of the present invention comprises or consists of SEQ ID NO:25. In some embodiments, the peptide of the present invention comprises or consists of SEQ ID NO:26. In some embodiments, the peptide of the present invention comprises or consists of SEQ ID NO:33. In some embodiments, the peptide of the present invention comprises or consists of SEQ ID NO:43.

The following are some embodiments of the TGF-β1 activation inhibiting peptide of the present invention.
1. A peptide having the amino acid sequence of formula (I) or a pharmaceutically acceptable salt, solvate or prodrug thereof:

   R¹-X¹-X²- X³-X⁴- X⁵- X⁶-X⁷-R² (I),

   wherein,
   R¹ is acetyl or absent;
   X¹ is absent or is a naturally occurring or non-naturally occurring amino acid residue;
   X² and X⁴ each independently are a naturally occurring or non-naturally occurring amino acid residue;
   X³ is D;
   X⁵ is E;
   X⁶ is D;
   X⁷ is a sequence consisting of 0 to 7 amino acids; and
   R² is amino or absent.
2. The peptide or the pharmaceutically acceptable salt, solvate, or prodrug thereof according to embodiment 1, wherein.
   X¹ is absent or is selected from homoserine, allothreonine, T, and S;
   X² is selected from naturally occurring amino acids or non-naturally occurring amino acids, preferably from polar side-chain amino acids, such as the uncharged polar side-chain amino acids S, C, G, N, Q, T, Y or negatively charged polar side-chain amino acids D and E, and more preferably selected from amino acids N, C, Q, S, T, E and D; and more preferably selected from amino acids Q, C, N, E and S;
   X³ is D;
   X⁴ is selected from small side-chain amino acids, such as A, C, G, P, S, T and V, more preferably selected from amino acids A, P and S, and more preferably selected from amino acids A and P;
   X⁵ is E;
   X⁶ is D;
   X⁷ is either absent or a sequence of 1, 2, 3, 4, 5, 6, or 7 amino acids, selected sequentially from the amino acid sequence of Z¹Z²Z³SZ⁴LQ, starting from the amino terminal to the carboxyl terminal,

   wherein Z¹, Z², Z³, Z⁴ are each independently selected from naturally occurring amino acids and non-naturally occurring amino acids, preferably:
      wherein Z¹ is an amino acid N, Q, P, A, L, V, M, or I, preferably N, P or L, most preferably N or P;
      Z² is an amino acid T, S, V, C, A, K, or R, preferably T, C, or K, more preferably, if Z² is an amino acid C or K, then Z² forms a covalent linkage with the side chain of amino acid X² or X³,
      Z³ is an amino acid A, V, L, or I, preferably A or V,
      Z⁴ is an amino acid F, Y, H, or W, preferably F or H;
   Optionally, if X⁴ is A, then 0 to 1 small side-chain amino acid, such as P or G, preferably P, is inserted between X³ and X⁴;
   Optionally, if X⁷ consists of 1 to 5 amino acids, then the peptide has 0 to 2 additional amino acid residues added after X⁷, e.g., 1 to 2 amino acid residues independently selected from Q, E, D and N, or 1 amino acid residue selected from C or K.
3. The peptide or the pharmaceutically acceptable salt, solvate or prodrug thereof according to embodiments 1-2, wherein X¹ is absent.
4. The peptide or the pharmaceutically acceptable salt, solvate or prodrug thereof according to embodiments 1-2, wherein X¹ is T or S.
5. The peptide or the pharmaceutically acceptable salt, solvate, or prodrug thereof according to embodiments 1-2, wherein X¹ is T.
6. The peptide or the pharmaceutically acceptable salt, solvate or prodrug thereof according to embodiments 1-5, wherein X² is selected from polar side chain amino acids.
7. The peptide or the pharmaceutically acceptable salt, solvate, or prodrug thereof according to embodiments 1-5, wherein X² is selected from uncharged polar side chain amino acids S, C, G, N, Q, T, and Y.
8. The peptide or the pharmaceutically acceptable salt, solvate or prodrug thereof according to embodiments 1-5, wherein X² is selected from negatively charged polar side chain amino acids D and E.
9. The peptide or the pharmaceutically acceptable salt, solvate, or prodrug thereof according to embodiments 1-5, wherein X² is selected from amino acids N, C, homoserine, T, allothreonine, E, and D.
10. The peptide or the pharmaceutically acceptable salt, solvate, or prodrug thereof according to embodiments 1-5, wherein X² is selected from amino acids Q, C, N, E, or S.
11. The peptide or the pharmaceutically acceptable salt, solvate or prodrug thereof according to embodiments 1-5, wherein X² is Q.
12. The peptide or the pharmaceutically acceptable salt, solvate or prodrug thereof according to embodiments 1-5, wherein X² is C.
13. The peptide or the pharmaceutically acceptable salt, solvate or prodrug thereof according to embodiments 1-5, wherein the side chain of X² forms a covalent linkage with the side chain of an amino acid comprised in X⁷, preferably both X² and Z² are C and the sulfhydryl groups of their side chains form a disulfide bond.
14. The peptide or the pharmaceutically acceptable salt, solvate, or prodrug thereof according to embodiments 1-13, wherein X⁴ is a small side chain amino acid, e.g., A, C, G, P, S, T, or V.
15. The peptide or the pharmaceutically acceptable salt, solvate, or prodrug thereof according to embodiments 1-13, wherein X⁴ is selected from amino acids A, P, and S.
16. The peptide or the pharmaceutically acceptable salt, solvate or prodrug thereof according to embodiments 1-13, wherein X⁴ is A.
17. The peptide or the pharmaceutically acceptable salt, solvate or prodrug thereof according to embodiments 1-13, wherein X⁴ is P.
18. The peptide or the pharmaceutically acceptable salt, solvate, or prodrug thereof according to embodiments 1-17, wherein X⁷ is absent.
19. The peptide or the pharmaceutically acceptable salt, solvate, or prodrug thereof according to embodiments 1-17, wherein X⁷ is a sequence consisting of 1, 2, 3, 4, 5, 6 or 7 amino acids sequentially from the amino terminal to the carboxyl terminal in the amino acid sequence of Z¹Z²Z³SZ⁴LQ.
20. The peptide or the pharmaceutically acceptable salt, solvate, or prodrug thereof according to embodiments 1-17, wherein X⁷ is a sequence consisting of 1, 2, 3, 4, or 5 amino acids sequentially from the amino terminal to the carboxyl terminal in the amino acid sequence of Z¹Z²Z³SZ⁴.
21. The peptide or the pharmaceutically acceptable salt, solvate or prodrug thereof according to embodiments 1-17, wherein X⁷ consists of Z¹.
22. The peptide or the pharmaceutically acceptable salt, solvate or prodrug thereof according to embodiments 1-17, wherein X⁷ consists of Z¹Z².
23. The peptide or the pharmaceutically acceptable salt, solvate, or prodrug thereof according to embodiments 1-17, wherein X⁷ consists of Z¹Z²Z³.
24. The peptide or the pharmaceutically acceptable salt, solvate, or prodrug thereof according to embodiments 1-17, wherein X⁷ consists of Z¹Z²Z³S.
25. The peptide or the pharmaceutically acceptable salt, solvate, or prodrug thereof according to embodiments 1-17, wherein X⁷ consists of Z¹Z²Z³S Z⁴.
26. The peptide or the pharmaceutically acceptable salt, solvate, or prodrug thereof according to embodiments 1-17, wherein X⁷ consists of Z¹Z²Z³S Z⁴L.
27. The peptide or the pharmaceutically acceptable salt, solvate or prodrug thereof according to embodiments 1-17, wherein X⁷ consists of Z¹Z²Z³S Z⁴LQ.
28. The peptide or the pharmaceutically acceptable salt, solvate, or prodrug thereof according to embodiments 1-27, wherein Z¹ is N, Q, P, A, N-methylalanine, L, norleucine, V, M, N-methylmethionine, or I.
29. The peptide or the pharmaceutically acceptable salt, solvate or prodrug thereof according to embodiments 1-27, wherein Z¹ is N.
30. The peptide or the pharmaceutically acceptable salt, solvate or prodrug thereof according to embodiments 1-27, wherein Z¹ is P.
31. The peptide or the pharmaceutically acceptable salt, solvate or prodrug thereof according to embodiments 1-27, wherein Z¹ is L.
32. The peptide or the pharmaceutically acceptable salt, solvate, or prodrug thereof according to embodiments 1-31, wherein Z² is T, allothreonine, S, homoserine, V, C, homocysteine, A, N-methylalanine, K, or R.
33. The peptide or the pharmaceutically acceptable salt, solvate, or prodrug thereof according to embodiments 1-31, wherein Z² is T.
34. The peptide or the pharmaceutically acceptable salt, solvate, or prodrug thereof according to embodiments 1-31, wherein Z² is C and is covalently linked to X² by the side chain.
35. The peptide or the pharmaceutically acceptable salt, solvate, or prodrug thereof according to embodiments 1-31, wherein Z² is K and is covalently linked to X³ by the side chain.
36. The peptide or the pharmaceutically acceptable salt, solvate, or prodrug thereof according to embodiments 1-35, wherein Z³ is A, N-methylalanine, V, L, norleucine, or I.
37. The peptide or the pharmaceutically acceptable salt, solvate, or prodrug thereof according to embodiments 1-35, wherein Z³ is A, particularly if Z¹ is N or P.
38. The peptide or the pharmaceutically acceptable salt, solvate, or prodrug thereof according to embodiments 1-35, wherein Z³ is V.
39. The peptide or the pharmaceutically acceptable salt, solvate or prodrug thereof according to embodiments 1-38, wherein Z⁴ is an aromatic amino acid.
40. The peptide or the pharmaceutically acceptable salt, solvate, or prodrug thereof according to embodiments 1-38, wherein Z⁴ is F, N-methylphenylalanine, homophenylalanine, Y, H, or W.
41. The peptide or the pharmaceutically acceptable salt, solvate, or prodrug thereof according to embodiments 1-38, wherein Z⁴ is F or H.
42. The peptide or the pharmaceutically acceptable salt, solvate, or prodrug thereof according to embodiments 1-38, wherein Z⁴ is H.
43. The peptide or the pharmaceutically acceptable salt, solvate, or prodrug thereof according to embodiments 1-42, wherein X⁷ consists of Z¹; or consists of Z¹Z²; or consists of Z¹Z²Z³ ; or consists of Z¹Z²Z³S; or consists of Z¹Z²Z³SZ⁴.
44. The peptide or the pharmaceutically acceptable salt, solvate or prodrug thereof according to embodiments 1-43, wherein X⁷ is an amino acid sequence selected from: N; P; L; NTA; NTV; PTA; NTAS; PTAS; NTVSF; NTASF; NTASH; NTVSFLQ.
45. The peptide or the pharmaceutically acceptable salt, solvate or prodrug thereof according to embodiments 1-44, wherein the peptide has 0-2 additional amino acid residues added after X⁷.
46. The peptide or the pharmaceutically acceptable salt, solvate or prodrug thereof according to embodiments 1-45, wherein the peptide comprises an amino acid sequence selected from: DAED; DPED; DAEDN; DPEDN; or DAEDP.
47. The peptide or the pharmaceutically acceptable salt, solvate or prodrug thereof according to embodiments 1-46, wherein X¹ is T and X² is Q.
48. The peptide or the pharmaceutically acceptable salt, solvate or prodrug thereof according to embodiments 1-47, wherein R¹ is acetyl.
49. The peptide or the pharmaceutically acceptable salt, solvate or prodrug thereof according to embodiments 1-48, wherein R² is amino.
50. A peptide having an amino acid sequence of the following general formula (II), or a pharmaceutically acceptable salt, solvate or prodrug thereof:

   R¹-X¹-X²-D-X⁴-E-D-X⁷-R² (II),

   wherein,
   R¹ is acetyl or absent;
   R² is amino or absent;
   X¹ is T or S;
   X² is selected from polar side-chain amino acids;
   X⁴ is selected from amino acids A or P;
   X⁷ is a sequence consisting of 1, 2, 3, 4, or 5 amino acids sequentially from the amino terminal to the carboxyl terminal in the amino acid sequence of Z¹TZ³SZ⁴,
   wherein:
   Z¹ is N, P or L;
   Z³ is A, V, L, or I,
   Z⁴ is F, Y, H, or W, preferably F or H.
51. The peptide or the pharmaceutically acceptable salt, solvate, or prodrug thereof according to embodiment 50, wherein X² is selected from amino acids Q, N, C, S, T, E, and D; preferably selected from Q, N, E, or S; more preferably X² is Q or N.
52. The peptide or the pharmaceutically acceptable salt, solvate, or prodrug thereof according to embodiment 50, wherein X² is Q.
53. The peptide or the pharmaceutically acceptable salt, solvate or prodrug thereof according to embodiments 50-52, wherein Z¹ is N, and Z³ is A or V; or Z¹ is P and Z³ is A.
54. The peptide or the pharmaceutically acceptable salt, solvate or prodrug thereof according to embodiments 50-53, wherein Z⁴ is F or H.
55. A peptide having an amino acid sequence of the following general formula (III) or a pharmaceutically acceptable salt, solvate, or prodrug thereof,

   R¹-X¹-cyclo(-C-D-X⁴-E-D-Z¹-C)-X⁸-R² (III),

   wherein,
   R¹ is acetyl or absent;
   R² is amino or absent;
   X¹ is T or S;
   X⁴ is selected from small side chain amino acids, such as A, C, G, P, S, T and V, more preferably selected from amino acids A, P and S, more preferably selected from amino acids A and P;
   Z¹ is a naturally occurring amino acid or a non-naturally occurring amino acid;
   X⁸ is absent or a sequence consisting of 1-5 amino acids.
56. The peptide or the pharmaceutically acceptable salt, solvate, or prodrug thereof according to embodiment 55, wherein X⁴ is A, C, G, P, S, T, or V.
57. The peptide or the pharmaceutically acceptable salt, solvate, or prodrug thereof according to embodiment 55, wherein X⁴ is A.
58. The peptide or the pharmaceutically acceptable salt, solvate, or prodrug thereof according to embodiment 55, wherein X⁴ is P.
59. The peptide, or the pharmaceutically acceptable salt, solvate, or prodrug thereof according to embodiments 55-58, wherein Z¹ is a polar uncharged amino acid or a non-polar amino acid.
60. The peptide or the pharmaceutically acceptable salt, solvate, or prodrug thereof according to embodiments 55-58, wherein Z¹ is N, Q, P, A, L, V, M, or I.
61. The peptide or the pharmaceutically acceptable salt, solvate, or prodrug thereof according to embodiments 55-58, wherein Z¹ is N.
62. The peptide or the pharmaceutically acceptable salt, solvate, or prodrug thereof according to embodiments 55-61, wherein X⁸ is absent.
63. The peptide, or the pharmaceutically acceptable salt, solvate, or prodrug thereof according to embodiments 55-61, wherein X⁸ is a sequence consisting of 1, 2, 3, 4, or 5 amino acids sequentially from the amino terminal to the carboxyl terminal in the amino acid sequence of Z³SZ⁴LQ, wherein Z³ is A, V, L, or I, and Z⁴ is F, Y, H, or W, preferably F or H.
64. The peptide or the pharmaceutically acceptable salt, solvate, or prodrug thereof according to embodiment 63, wherein Z³ is A or V, and Z⁴ is F or H.
65. The peptide or the pharmaceutically acceptable salt, solvate or prodrug thereof according to embodiments 55-64, wherein the peptide consists of 5 to 13 amino acids, preferably consists of 7, 8, 9, 10 or 11 amino acids.
66. The peptide or the pharmaceutically acceptable salt, solvate or prodrug thereof according to embodiments 1-54, wherein the peptide consists of 5 to 13 amino acids, preferably consists of 7, 8, 9, 10 or 11 amino acids.
67. The peptide or the pharmaceutically acceptable salt, solvate or prodrug thereof according to embodiment 66, wherein the peptide is a linear peptide.
68. The peptide or the pharmaceutically acceptable salt, solvate or prodrug thereof according to embodiment 66, wherein the peptide is a cyclic peptide, and preferably, the cyclization region of the peptide comprises a core motif (DXED).
69. The peptide or the pharmaceutically acceptable salt, solvate or prodrug thereof according to embodiment 68, wherein the peptide is head-to-tail cyclized or side-chain to side-chain cyclized.
70. The peptide or the pharmaceutically acceptable salt, solvate or prodrug thereof according to embodiment 68, wherein the peptide is head-to-tail cyclized.
71. The peptide or the pharmaceutically acceptable salt, solvate or prodrug thereof according to embodiment 68, wherein the peptide is side-chain to side-chain cyclized.
72. The peptide or the pharmaceutically acceptable salt, solvate, or prodrug thereof according to embodiment 68, wherein the peptide is cyclized through coupling side chains of amino acids at positions 3 and 8, or through coupling side chains of amino acids at positions 2 and 8.
73. The peptide or the pharmaceutically acceptable salt, solvate or prodrug thereof according to embodiments 71-72, wherein the peptide is cyclized through a lactam bond formed between amino acid side chains.
74. The peptide or the pharmaceutically acceptable salt, solvate, or prodrug thereof according to embodiments 71-72, wherein the peptide is cyclized through a disulfide bond formed between amino acid side chains.
75. The peptide or the pharmaceutically acceptable salt, solvate or prodrug thereof according to embodiments 71-74, wherein the peptide is cyclized through side chains,
   wherein X³ is D, Z² is K, and the peptide is cyclized through a lactam bond formed between the side chains of X³ and Z²; or
   wherein X² is C, Z² is C, and the peptide is cyclized through a disulfide bond formed between the side chains of X² and Z²;
   Preferably, if Z² is amino acid C or K, then X⁷ consists of Z¹Z², and X¹ is T or S.
76. A peptide or a pharmaceutically acceptable salt, solvate, or prodrug thereof, wherein the peptide has an amino acid sequence corresponding to any one selected from SEQ ID NOs: 1 to 49, or comprises or consists of any one selected from SEQ ID NOs: 1 to 49.
77. A peptide or a pharmaceutically acceptable salt, solvate, or prodrug thereof, wherein the peptide has an amino acid sequence corresponding to any one selected from SEQ ID NOs: 6, 22, 25, 26, 33, 40, 43, 44, and 46, or comprises or consists of any one selected from SEQ ID NOs: 6, 22, 25, 26, 33, 40, 43, 44, and 46.
78. A peptide or a pharmaceutically acceptable salt, solvate or prodrug thereof, wherein the peptide comprises or consists of SEQ ID NO: 6.
79. A peptide or a pharmaceutically acceptable salt, solvate, or prodrug thereof, wherein the peptide comprises or consists of SEQ ID NO: 22.
80. A peptide or a pharmaceutically acceptable salt, solvate, or prodrug thereof, wherein the peptide comprises or consists of SEQ ID NO: 25.
81. A peptide or a pharmaceutically acceptable salt, solvate, or prodrug thereof, wherein the peptide comprises or consists of SEQ ID NO: 26.
82. A peptide or a pharmaceutically acceptable salt, solvate, or prodrug thereof, wherein the peptide comprises or consists of SEQ ID NO: 33.
83. A peptide or a pharmaceutically acceptable salt, solvate, or prodrug thereof, wherein the peptide comprises or consists of SEQ ID NO: 40.
84. A peptide or a pharmaceutically acceptable salt, solvate, or prodrug thereof, wherein the peptide comprises or consists of SEQ ID NO: 43.
85. A peptide or a pharmaceutically acceptable salt, solvate, or prodrug thereof, wherein the peptide comprises or consists of SEQ ID NO: 44.
86. A peptide or a pharmaceutically acceptable salt, solvate, or prodrug thereof, wherein the peptide comprises or consists of SEQ ID NO: 46.
87. A peptide or a pharmaceutically acceptable salt, solvate or prodrug thereof, wherein the peptide has 1, 2, 3, 4 or 5 amino acid differences, preferably 1 or 2 amino acid substitutions, deletions or additions, as compared to the peptide according to one of embodiments 76-86, and preferably the substitution occurs at an amino acid residue other than the core motif (DXED), e.g., for conservative amino acid substitution.
88. The peptide or the pharmaceutically acceptable salt, solvate, or prodrug thereof according to embodiments 1-87, wherein the peptide is chemically modified.
89. The peptide or the pharmaceutically acceptable salt, solvate or prodrug thereof according to embodiments 1-88, wherein the peptide comprises a PEG modification.
90. The peptide or the pharmaceutically acceptable salt, solvate or prodrug thereof according to embodiments 1-89, wherein the peptide comprises a lipid modification.
91. The peptide or the pharmaceutically acceptable salt, solvate or prodrug thereof according to embodiments 1-90, wherein the peptide comprises a D-type amino acid substitution and/or a non-naturally occurring amino acid substitution.

### Method for the preparation of the inhibitory peptide of the present invention

In an aspect, the present invention provides a method for the preparation of the inhibitory peptide of the present invention. The preparation of the peptide of the present invention can be carried out using a chemical synthesis method, fermentation, or a genetic recombination technique.

The method for chemically synthesizing a peptide is well known in the field and includes solid phase synthesis and liquid phase synthesis techniques. The desired peptide chain can be synthesized using a solid phase polypeptide synthesis (SPPS) technique developed by Merrifield. Automated peptide synthesizer can also be used, to perform the synthesis. Subsequent purification of a SPPS peptide product is simpler and easier than a recombinantly synthesized peptide. Therefore, it is preferred to apply the SPPS technique to synthesize the short peptide of the present invention.

SPPS technology generally consists of cycles of coupling and deprotection. In Fmoc synthesis strategy, in the coupling step, Fmoc-protected amino acid (Fmoc-AA-OH) is coupled to a solid polymer resin by applying a condensing agent such as HBTU/HATU/DIC, etc. In the deprotection step, the protecting group Fmoc is removed from the amino acid by means of a deprotecting agent, such as piperidine, etc., to release amino groups, which then proceed to the next coupling and deprotection cycle. The whole process can be performed in a sieve reactor till the final peptide is synthesized and cleaved off from the resin. Afterwards, the synthesized peptide product can be purified and detected by high performance liquid chromatography.

A variety of resins, such as 4-methylbenzhydrylamine (HMBA) resin, Wang resin, 2-chlorotrityl chloride (CTC) resin, and Merrifield resin, can be used for solid-phase synthesis of a peptide. In addition, a variety of functional resins have also been developed by coupling a resin to different linkers, to allow peptide cyclization in the solid phase.

In an embodiment, the present invention provides a method for the preparation of a peptide of the present invention, comprising: (1) synthesizing a linear peptide having a defined sequence, e.g., by using 4-methylbenzhydrylamine (HMBA) resin as the starting material, Fmoc-protected amino acids as monomers, and hexahydropyridine/DMF solution as the deprotecting reagent, and sequentially linking amino acids one by one in the presence of a condensing agent and under alkaline condition to produce the linear peptide; (2) optionally, cyclizing the linear peptide obtained in step (1); (3) purifying the peptide product obtained in step (1) or (2).

In another embodiment, the present invention provides a method for the preparation of a peptide of the present invention, comprising (1) recombinantly expressing a linear peptide having a defined sequence; (2) optionally, cyclizing the linear peptide obtained in step (1); and (3) purifying the peptide product obtained in step (1) or (2). In some aspects, the present invention also provides a nucleic acid encoding the amino acid sequence of a peptide of the present invention, and a vector (e.g., an expression vector) and a host cell comprising the nucleic acid.

Peptide cyclization is a common peptide modification technique that includes a variety of strategies, such as head-to-tail cyclization, side-chain-to-side-chain cyclization, and backbone -to-side-chain cyclization. Typically, a single linear peptide is too flexible if it has not linked to other peptides. In contrast, cyclization, by organizing intramolecular interactions, can promote the formation of a secondary structure in a peptide, thereby improve the stability of the peptide.

In one embodiment according to the present invention, the peptide of the present invention forms a monocyclic peptide through head-to-tail cyclization. In another embodiment according to the present invention, the peptide of the present invention, through side-chain cyclization, forms a lactam bridge between the side chains of glutamic acid (E) or aspartic acid (D) and lysine (K). In another embodiment according to the present invention, the peptide of the present invention, through side-chain cyclization, forms a disulfide bond between the side chains of two cysteine (C) residues. In embodiments of the cyclic peptide of the present invention comprising a lactam bridge, preferably a lysine amino acid residue is located at the C-terminal of the core sequence (DXED) of the present invention, and forms the lactam bridge with the first residue aspartic acid D of the core sequence (DXED); more preferably the K residue is at least 4 residues, e.g., 4, 5, or 6 residues apart from the D residue, preferably the lysine residue is the last residue at the C-terminal of the peptide of the present invention. In embodiments of the cyclic peptide of the present invention comprising a disulfide bond, preferably the two cysteine residues for cyclization are located at the N-terminal and the C-terminal of the core sequence (DXED) respectively, and more preferably the two cysteine residues are separated by at least 5 residues, e.g., 5, 6, 7, 8 residues. In a preferred embodiment, the amino acid at position 3 of the peptide of the present invention is D and the amino acid at position 8 is K, and the peptide of the present invention is cyclized by the formation of a lactam bond through their side chains. In another preferred embodiment, the amino acid at position 2 of the peptide of the present invention is C and the amino acid at position 8 is C, and the peptide of the present invention is cyclized by the formation of a disulfide bond through their side chains.

After the peptide is synthesized, it may be modified, with or without the use of medicinal chemistry techniques. In some instances, modifying a peptide may be advantageous, for example, by modifying to mimic, stabilize, or construct a more suitable secondary structure to improve the biological activity of the peptide drug, and/or to improve the selectivity, stability, and solubility of the peptide drug. Peptide modification may be amino acid substitutions or residue modifications of non-essential amino acids, and/or may be modification at the N-terminal and/or C-terminal of the peptide, such as N-terminal acetylation and C-terminal amidation. Modification may also be a substitution of a critical amino acid residue that affects the biological activity of the peptide, in order to seek a change in activity.

In some instances, the peptide may comprise a non-naturally occurring amino acid. If necessary, the peptide may comprise 1, 2, 3, 4, 5, or 6, or more non-naturally occurring amino acids. Alternatively, the amino acids comprised in the peptide may each be independently selected from naturally occurring amino acids.

Chemically synthesized peptides often carry free amino group and free carboxyl group. In some instances, the ends of the peptides can be blocked, i.e., N-terminal acetylation and C-terminal amidation, to make the synthetic peptides as mimics closer to natural proteins and to improve the stability of the peptides.

Thus, in some aspects, the peptide of the present invention may comprise an D-amino acid, a non-naturally occurring amino acid, an amino acid analog, and/or group substitution and modification; or may be linked to a polymer or to a drug carrier.

### Pharmaceutical compositions

In an aspect, the present invention also provides a pharmaceutical composition comprising a peptide of the present invention or a pharmaceutically acceptable salt, solvate or prodrug thereof. As is well understood in the art, the pharmaceutical composition may optionally further comprise suitable pharmaceutical auxiliary materials, pharmaceutical carriers, pharmaceutical excipients, including buffering agents.

Administration routes for peptide delivery include, but are not limited to, subcutaneous administration, intramuscular administration, and intravenous administration, mucosal administration (e.g., nasal administration, pulmonary mucosal administration, sublingual administration), oral administration (e.g., by addition of a gastrointestinal pro-osmotic agent or carrier), and transdermal administration. The peptide can be formulated into any formulation suitable for administration, such as an injection, e.g., an intravenous injection or intravenous infusion, a lyophilized powder, and the like. Auxiliary materials for use in different formulation forms are known in the art.

As is well understood in the art, the pharmaceutical composition may further comprise another therapeutic agent that is beneficial for the specific disease to be treated. **In** some embodiments, therefore, the present invention also provides a pharmaceutical composition comprising the peptide of the present invention or a pharmaceutically acceptable salt, solvate, or prodrug thereof, and also comprising another therapeutic agent that is beneficial for the specific disease to be treated. In such embodiments, the peptide of the present invention may be comprised in the same or different composition as the other therapeutic agent; and may be administered with the other therapeutic agent concurrently, sequentially, or in any order, and using any dosing regimen.

In some embodiments, the pharmaceutical composition of the present invention comprises, in particular, the peptide of the invention or a pharmaceutically acceptable salt thereof of the preceding embodiments 1-91, in particular the peptide of the invention or a pharmaceutically acceptable salt thereof of one of the preceding embodiments 76-86, and in particular, the peptide of the invention or a pharmaceutically acceptable salt thereof of one of the preceding embodiments 78-86.

### Treatment methods and uses

In an aspect, the present invention provides the peptide of the invention or a pharmaceutically acceptable salt, solvate, or prodrug thereof, or a pharmaceutical composition of the invention for use as a therapy in preventing or treating a TGF-β related disease in a subject, and a method for treatment and prevention of the said disease.

As used herein, the term "TGF-β related disease" refers to diseases and disorders associated with, and/or resulting from, TSP-1-involved TGF-β1 activation or pathologic increases in TGF-β1 activity, including, but not limited to, fibrosis, chronic inflammation, and cancer. The TGF-β related disease would benefit from an inhibitory effect on TSP-1 involved TGF-β1 activation.

As used herein, the term "subject" or "patient" or "individual" includes any human or non-human animal. The term "nonhuman animal" includes all vertebrates, such as mammals and non-mammals, such as non-human primates, sheep, dogs, cats, horses, cows, chickens, amphibians, reptiles and the like. Preferably, the subject according to the present invention is a human.

As used herein, the term "effective amount" or "effective dosage" refers to the amount of a peptide of the invention, or a pharmaceutically acceptable salt, solvate, or prodrug thereof, which, when administered to cells, tissues, or subjects, alone or in combination with other therapeutic agent(s), is effective in preventing or ameliorating the symptom(s) of one or more diseases or disorders, or the progression of the diseases or disorders. An effective dose also refers to an amount sufficient to improve a symptom, such as an amount sufficient to treat, cure, prevent, or ameliorate a relevant medical condition, or increases the rate of treatment, cure, prevention, or amelioration of the condition. When an active ingredient is administered alone to an individual, the effective dose refers only to that ingredient. When administered in combination, the effective amount refers to the combined amount of active ingredients that cause a therapeutic or preventive effect, regardless of administered concurrently, sequentially, or simultaneously. In some embodiments, the effective amount will result in at least 10%, typically at least 20%, preferably at least about 30%, more preferably at least 40%, and most preferably at least 50% improvement in a diagnostic criterion or parameter.

In some embodiments according to the present invention, "treatment" of a disease or disorder means ameliorating the disease or disorder (i.e., slowing or inhibiting or reducing the progression of the disease or at least one of its clinical symptoms). In some other embodiments, "treatment" refers to alleviating or improving at least one physical parameter, including those physiological parameters that may not be discernible by the patient. In some other embodiments, "treatment" refers to modifying the disease or disorder physically (e.g., stabilization of a discernible symptom), physiologically (e.g., stabilization of a physical parameter), or both. Methods for evaluating the treatment and/or prevention of a disease are generally known in the art, unless expressly described herein otherwise.

In further embodiments according to the present invention, "prevention" of a disease or disorder comprises inhibition of the onset or progression of the disease or disorder or a particular symptom of the disease or disorder. Typically, in the context of fibrosis or cancer, the term "prevention" refers to the administration of a drug prior to the onset of signs or symptoms of fibrosis or cancer, particularly in subjects at risk of developing the disease.

Accordingly, in some embodiments, the present invention provides methods for preventing or treating a TGF-β related disease, comprising administering to a subject in need thereof an effective amount of the peptide according to the present invention or a pharmaceutically acceptable salt, solvate, or prodrug thereof or the pharmaceutical composition according to the present invention. Optionally, the method further comprises administering to the subject an effective amount of a second therapeutic agent, such as a chemotherapeutic agent or an immunotherapeutic agent. Preferably, the TGF-β related disease to be treated according to the method of the present invention has macrophage infiltration at the lesion site. In some embodiments, the TGF-β related disease is associated with tissue damage, inflammation or fibrosis. In some embodiments, the TGF-β related disease involves metastasis of tumor cells.

In some embodiments according to the prophylactic or therapeutic method of the invention, the peptide of the invention or a pharmaceutically acceptable salt thereof of the preceding embodiments 1-91, in particular the peptide of the invention or a pharmaceutically acceptable salt thereof of one of the preceding embodiments 76-86, and in particular, the peptide of the invention or a pharmaceutically acceptable salt thereof of one of the preceding embodiments 78-86, may be particularly used.

### Treatment of fibrotic disorder

In some embodiments, the disorder associated with a pathological increase in TGF-β activity is fibrosis. In one embodiment, the present invention provides the peptide of the present invention or a pharmaceutically acceptable salt, solvate, or prodrug thereof, or a pharmaceutical composition of the present invention for use as a therapy in the prevention or treatment of a fibrotic disorder in a subject, and the corresponding therapeutic and prophylactic method.

A wide range of diseases are now recognized to exhibit fibrotic features. These diseases may be classified based on the tissues and organs affected by fibrosis, such as the lungs, liver, kidneys, bone marrow, and other tissues, or categorized under general conditions, e.g., chronic inflammatory diseases, tumor diseases, and immune disorders. The presence of fibrosis as a pathological feature is becoming a critical factor in redefining, and even naming, diagnosing, and treating fibrotic disorders, moving beyond traditional classifications and the limitations of disease location-based knowledge.

As used herein, therefore, "fibrotic disorder" refers to a disease characterized by a fibrotic pathological phenotype, and is not limited to a specific site of disease occurrence or a conventional disease classification. Examples of fibrotic disorders that may be treated by the methods of the present invention include, but are not limited to, fibrosis of various tissues and organs, e.g., pulmonary fibrosis, hepatic fibrosis, renal fibrosis; as well as chronic inflammatory, tumor, and immune disorders.

In some embodiments, therefore, the present invention provides a method for preventing or treating a fibrotic disorder in a subject, comprising administering to a subject in need thereof a prophylactically or therapeutically effective amount of the peptide according to the present invention or a pharmaceutically acceptable salt, solvate, or prodrug thereof, or the pharmaceutical composition according to the present invention. In some embodiments, the fibrotic disorder is hepatic fibrosis, pulmonary fibrosis, renal fibrosis, myelofibrosis, dermal fibrosis, or cardiac fibrosis.

In some embodiments, the fibrotic disorder is pulmonary fibrosis, e.g., idiopathic pulmonary fibrosis (IPF). In some embodiments, the method comprises administering the peptide of the invention to the subject by inhalation, e.g., as a nebulized formulation.

The therapeutic uses and methods described herein may also include co-formulating and/or co-administering the peptide of the invention or a pharmaceutically acceptable salt, solvate, or prodrug or a pharmaceutical composition thereof with another therapeutically effective agent for the prevention and/or treatment of a pathological fibrotic disease.

### Treatment of tumor

In some embodiments, the disorder associated with a pathological increase in TGF-β activity is cancer. In one embodiment, the present invention provides the peptide of the present invention or a pharmaceutically acceptable salt, solvate, or prodrug or pharmaceutical composition thereof for use as a therapy in the prevention or treatment of a cancer in a subject, and the corresponding therapeutic and prophylactic method. In some embodiments, the cancer is a solid tumor, preferably selected from: lung cancer, liver cancer, breast cancer, uterine cancer, prostate cancer, pancreatic cancer, colon cancer, skin cancer, central nervous system cancer, fibromyoma, fibroma, fibroadenoma, and fibrosarcoma. In some embodiments, the cancer is sarcoma, pancreatic cancer, glioblastoma, head and neck cancer, melanoma, breast cancer, or colorectal cancer. In some embodiments, the cancer is selected from squamous cell carcinoma, epidermoid carcinoma, uroepithelial carcinoma, adenocarcinoma, adrenocortical carcinoma, basal cell carcinoma, ductal carcinoma in situ (DCIS), invasive ductal carcinoma, thymic carcinoma, and renal cell carcinoma.

In some embodiments, the method comprises administering the peptide of the present invention or a pharmaceutically acceptable salt, solvate, or prodrug or a pharmaceutical composition thereof to a subject via an oral, intravenously, intratumorally, transdermally, subcutaneously, or locally. In some embodiments, the method comprises contacting a cancerous tissue (e.g., cancerous skin tissue) of the subject with a formulation comprising a therapeutically effective amount of the peptide of the present invention or a pharmaceutically acceptable salt, solvate, or prodrug or a pharmaceutical composition thereof for a period of time sufficient to treat the cancer.

The therapeutic uses and methods described herein may also include co-formulating and/or co-administering the peptide of the invention or a pharmaceutically acceptable salt, solvate, or prodrug or a pharmaceutical composition thereof with another therapeutically effective agent for the prevention and/or treatment of cancer.

### Other uses

In some aspects, the present invention also provides the uses of the peptide of the invention or a pharmaceutically acceptable salt, solvate, or prodrug thereof for:
- blocking the binding of CD36 to TSP-1;
- inhibiting TSP-1-dependent TGF-β1 activation;
- decreasing the amount of active TGF-β1 in fibrotic tissue;
- inhibiting TGF-β1-mediated downstream signaling;
- reducing collagen deposition in fibrotic tissue;
- inhibiting inflammatory and/or fibrotic lesions mediated by TGF-β1;
- inhibiting extracellular matrix-related gene expressions stimulated by TGF-β1;
- inhibiting tumor cell migration;
- preventing or treating TGF-β1 related diseases, especially fibrosis or cancer; or
in the manufacture of a medicament for the above uses.

The above uses may be in vitro or in vivo. In a preferred embodiment, the use is an *in vivo* use for treatment or prevention of a disease. A method for the *in vivo* use comprises administering the peptide of the invention or a pharmaceutically acceptable salt, solvate or prodrug thereof, optionally in combination with another therapeutic agent(s), in a therapeutically effective amount.

For the prevention or treatment of a disease, an appropriate dosage of the peptide of the present invention or a pharmaceutically acceptable salt, solvate, or prodrug thereof (when used alone or in combination with one or more other therapeutic agents) depends on the type of disease to be treated, the type of the specific medication, the severity and course of the disease, whether administered for prophylactic purposes or for therapeutic purposes, previous therapy, the patient's clinical history, and response to the medication, and the judgment of the attending physician. The drug may be appropriately administered to the patient as a single treatment or over a series of treatments.

In a further aspect, the present invention also provides a use of the peptide of the present invention or a pharmaceutically acceptable salt, solvate, or prodrug thereof in the manufacture of a medicament for use in the foregoing methods (e.g., for therapeutic use).

The following Examples are provided to aid in the understanding of the present invention. It should be understood, however, that these Examples are not intended to, and should not, constitute a limitation on the protection scope of the present invention in any way.

For the purposes of the present invention, when referring to amino acid residues herein, the following amino acid codes, including three-letter and single-letter codes, are appliable.

| Amino acids | Three-letter code | Single-letter code |
|---|---|---|
| L-Alanine | Ala | A |
| L-Valine | Val | V |
| L-Leucine | Leu | L |
| L-Isoleucine | Ile | I |
| L-Phenylalanine | Phe | F |
| L-Histidine | His | H |
| L-Tyrosine | Tyr | Y |
| L-Tryptophan | Trp | W |
| L-Lysine | Lys | K |
| L-Arginine | Arg | R |
| L-Aspartic acid | Asp | D |
| L-Asparagine | Asn | N |
| L-Glutamic acid | Glu | E |
| L-Glutamine | Gln | Q |
| L-Proline | Pro | P |
| L-Cysteine | Cys | C |
| L-Serine | Ser | S |
| L-Threonine | Thr | T |
| L-Methionine | Met | M |
| Glycine | Gly | G |
| Any amino acid | Xaa | X |

### Examples

### Example 1. Polypeptide preparation

The polypeptides used in Examples of the invention were prepared according to Fmoc (9-fluorenylmethoxycarbonyl) solid-phase synthesis. Taking the carboxy-terminal amino acid of a linear peptide as the starting point for synthesis, in a polypeptide solid-phase synthesis reaction column, a Rink Amide MBHA Resin (Gill Biochemistry, 49101) was firstly activated by soaking in DCM solution for 30 min and then pumped dry, and 20% of hexahydropyridine + 80% of DMF solution was added with stirring and agitating under nitrogen gas for 30 min to carry out a deprotection reaction. The synthesis reaction was carried out by feeding Fmoc-protected amino acids, condensing agent and organic bases. After the reaction, the ninhydrin colorimetric method was performed to detect whether the reaction was complete. According to the amino acid sequence (from carboxyl-terminal to amino-terminal) of each peptide, the above feeding, reaction and detection steps were repeated until the last amino acid, after which a desired peptide was cleaved from the resin to obtain as a crude product.

A cyclized peptide was prepared as follows:
(1) Disulfide cyclization: A linear polypeptide synthesized and cleaved from the resin as described above was cyclized in an aqueous solution (peptide concentration of 1g/L) at pH=7.5-8 under stirring for more than 12h.
(2) Side-chain cyclization: During the aforementioned linear polypeptide synthesis, special side-chain protecting groups, such as D (oall) and K (alloc), were selected to protect the side-chain carboxyl group of D and the side-chain amino group of K. At the end of the linear polypeptide sequence synthesis, the -oall/-alloc were removed from the resin, and cyclization was carried out using a corresponding condensing agent.
(3) Head-to-tail cyclization: A linear polypeptide synthesized and cleaved from the resin as described above was prepared into a fully protected polypeptide fragment, and a corresponding condensing agent in DMF or other solvent was added for cyclization. At the end of the reaction, a product was decanted from the water and dried, and the protective groups on the cyclic peptide were removed to yield a de-protected and head-to-tail cyclized polypeptide as a crude product.

The synthesized linear and cyclic crude peptides were separated and purified by HPLC on a C18 column, using 0.1% TFA/acetonitrile solution as eluent A and 0.1% TFA/water solution as eluent B. The fractions from the main peaks were collected, and after the products were detected by mass spectrometry and found to have a molecular weight matching the theoretical value, they were lyophilized, yielding refined peptides. The sequences of the synthesized polypeptides were shown in Table 1, in which peptides represented by each SEQ ID NOs consist of the "Sequence" and the "Modification" provided in the sequence description.

**Table 1. Sequence listing of polypeptides**

| SEQ ID NO: | Sequence | Modification |
|---|---|---|
| **SEQ ID NO:01** | QDPED | N-terminal acetylation, C-terminal amidation |
| **SEQ ID NO:02** | QDAED | N-terminal acetylation, C-terminal amidation |
| **SEQ ID NO:03** | TQDPED | N-terminal acetylation, C-terminal amidation |
| **SEQ ID NO:04** | TQDAED | N-terminal acetylation, C-terminal amidation |
| **SEQ ID NO:05** | QDAEDN | N-terminal acetylation, C-terminal amidation |
| **SEQ ID NO:06** | TQDAEDN | N-terminal acetylation, C-terminal amidation |
| **SEQ ID NO:07** | SQDAEDN | N-terminal acetylation, C-terminal amidation |
| **SEQ ID NO:08** | TNDAEDN | N-terminal acetylation, C-terminal amidation |
| **SEQ ID NO:09** | TEDAEDN | N-terminal acetylation, C-terminal amidation |
| **SEQ ID NO:10** | TSDAEDN | N-terminal acetylation, C-terminal amidation |
| **SEQ ID NO:11** | TQDSEDN | N-terminal acetylation, C-terminal amidation |
| **SEQ ID NO:12** | TQDAEDL | N-terminal acetylation, C-terminal amidation |
| **SEQ ID NO:13** | TQDAEDP | N-terminal acetylation, C-terminal amidation |
| **SEQ ID NO:14** | TQDPEDN | N-terminal acetylation, C-terminal amidation |
| **SEQ ID NO:15** | TQDAEDNT | N-terminal acetylation, C-terminal amidation |
| **SEQ ID NO:16** | QDAEDNTA | N-terminal acetylation, C-terminal amidation |
| **SEQ ID NO:17** | TQDAEDNTV | N-terminal acetylation, C-terminal amidation |
| **SEQ ID NO:18** | SQDAEDNTV | N-terminal acetylation, C-terminal amidation |
| **SEQ ID NO:19** | TNDAEDNTV | N-terminal acetylation, C-terminal amidation |
| **SEQ ID NO:20** | TQDPEDNTV | N-terminal acetylation, C-terminal amidation |
| **SEQ ID NO:21** | TQDSEDNTV | N-terminal acetylation, C-terminal amidation |
| **SEQ ID NO:22** | TQDAEDPTA | N-terminal acetylation, C-terminal amidation |
| **SEQ ID NO:23** | QDAEDNTAS | N-terminal acetylation, C-terminal amidation |
| **SEQ ID NO:24** | TQDAEDNTAS | N-terminal acetylation, C-terminal amidation |
| **SEQ ID NO:25** | TQDAEDPTAS | N-terminal acetylation, C-terminal amidation |
| **SEQ ID NO:26** | TQDPEDNTAS | N-terminal acetylation, C-terminal amidation |
| **SEQ ID NO:27** | TQDAEDNTVSF | N-terminal acetylation, C-terminal amidation |
| **SEQ ID NO:28** | SQDAEDNTVSF | N-terminal acetylation, C-terminal amidation |
| **SEQ ID NO:29** | TEDAEDNTVSF | N-terminal acetylation, C-terminal amidation |
| **SEQ ID NO:30** | TQDPEDNTVSF | N-terminal acetylation, C-terminal amidation |
| **SEQ ID NO:31** | TQDAEDNTASF | N-terminal acetylation, C-terminal amidation |
| **SEQ ID NO:32** | TQDAEDNTASY | N-terminal acetylation, C-terminal amidation |
| **SEQ ID NO:33** | TQDAEDNTASH | N-terminal acetylation, C-terminal amidation |
| **SEQ ID NO:34** | TQDAEDNTASW | N-terminal acetylation, C-terminal amidation |
| **SEQ ID NO:35** | TQDAEDNTASHL | N-terminal acetylation, C-terminal amidation |
| **SEQ ID NO:36** | TQDAEDNTVSFLQ | N-terminal acetylation, C-terminal amidation |
| **SEQ ID NO:37** | SQDAEDNTVSFLQ | N-terminal acetylation, C-terminal amidation |
| **SEQ ID NO:38** | TSDAEDNTVSFLQ | N-terminal acetylation, C-terminal amidation |
| **SEQ ID NO:39** | TQDPEDNTVSFLQ | N-terminal acetylation, C-terminal amidation |
| **SEQ ID NO:40** | TQDAEDN | Head-to-tail cyclization |
| **SEQ ID NO:41** | TQDPEDN | Head-to-tail cyclization |
| **SEQ ID NO:42** | TQDAEDNK | Cyclization of D(3) K(8) side-chains |
| **SEQ ID NO:43** | TCDAEDNC | N-terminal acetylation, disulfide Cyclization |
| **SEQ ID NO:44** | TCDAEDNC | N-terminal acetylation, C-terminal amidation, disulfide Cyclization |
| **SEQ ID NO:45** | QDAEDNT | Head-to-tail cyclization |
| **SEQ ID NO:46** | TQDPEDNTAS | Head-to-tail cyclization |
| **SEQ ID NO:47** | TQDPAED | N-terminal acetylation, C-terminal amidation |
| **SEQ ID NO:48** | TQDPAEDN | N-terminal acetylation, C-terminal amidation |
| **SEQ ID NO:49** | TQDAEDNTQD | N-terminal acetylation, C-terminal amidation |
| **SEQ ID NO:50** | YRVRFLAKENVTQDAEDN | Without modification |

### Example 2. In vitro target blocking activity assay of polypeptides

ELISA assay was performed to detect the ability of the polypeptides to block the binding of TSP-1 protein to CD36, a TSP-1 receptor protein. TSP-1 protein (novoprotein, Catalog No. CU45) was diluted to 2 µg/mL using 100 mM carbonate buffer and added to sample wells (100 µL/well) of a 96-well ELISA plate (Thermo, Catalog No. 437111), and incubated overnight at 4°C; after washing the plate twice using 1 x PBST wash solution, 200 µL of 1.5% BSA (Sigma, Catalog No. B2064) was added to each well, and incubated at 37°C for 2 h; after washing the plate 3 times using 1 x PBST wash solution, the analytes were added (100 µL/well, 3 replicate wells; for the control group, 0.25 µg/mL of Fc-tagged CD36 protein (R&D Systems, Catalog No. 1955-CD-050); and for the polypeptide-treated groups, a mixture containing Fc-tagged CD36 protein and polypeptide at final concentrations of 0.25 µg/mL and 100 µM, respectively), and incubated at 37°C for 3 h. After washing the plate 5 times using 1x PBST wash solution, a 1:500 dilution solution of FITC-labeled anti-Fc-tagged antibody (Beyotime, Catalog No. A0556) was added, and incubated for 1 h at 37°C; after washing the plate 5 times using 1x PBST wash solution, a multifunctional ELISA instrument (TECAN, model: Infinite 200 Pro) was used to detect the intensity of FITC fluorescence signal in each sample well. The ability of the polypeptides to block CD36 binding to TSP-1 protein (Table 2-1) was assessed by calculating the ratio of the fluorescence signal from the polypeptide-treated groups to that of the control group, allowing for the determination of the blocking rate of the polypeptides in inhibiting CD36 binding to TSP-1 protein, i.e., (fluorescence signal of the control group - fluorescence signal of the polypeptide-treated group)/fluorescence signal of the control group × 100%. Based on the blocking rate results obtained at the single concentration and on the polypeptide molecule properties, the preferred polypeptide molecules were assayed for concentration dependence (at 0 µM, 0.5 µM, 1 µM, 5 µM, 10 µM, and 100 µM), and IC₅₀ values of the polypeptides for blocking CD36 binding to TSP-1 protein were determined to further assess the abilities of the preferred polypeptide molecules to block the binding of CD36 to TSP-1 (Table 2-2). The assay results showed that all the polypeptides of the present invention inhibit the binding of CD36 to TSP-1.

**Table 2-1 In vitro target binding assay data of polypeptides**

| **SEQ ID NO:** | **Blocking rate (Mean, %)** | **±SD (%)** | **SEQ ID NO:** | **Blocking rate (Mean, %)** | **±SD (%)** |
|---|---|---|---|---|---|
| **SEQ ID NO:01** | 17.84 | 1.96 | **SEQ ID NO:26** | 53.77 | 2.28 |
| **SEQ ID NO:02** | 16.39 | 1.56 | **SEQ ID NO:27** | 25.53 | 1.88 |
| **SEQ ID NO:03** | 16.02 | 1.28 | **SEQ ID NO:28** | 21.71 | 1.46 |
| **SEQ ID NO:04** | 16.89 | 1.34 | **SEQ ID NO:29** | 25.05 | 0.76 |
| **SEQ ID NO:05** | 15.07 | 0.78 | **SEQ ID NO:30** | 22.45 | 0.97 |
| **SEQ ID NO:06** | 17.74 | 1.14 | **SEQ ID NO:31** | 18.44 | 2.62 |
| **SEQ ID NO:07** | 16.42 | 1.20 | **SEQ ID NO:32** | 21.45 | 2.08 |
| **SEQ ID NO:08** | 16.70 | 2.33 | **SEQ ID NO:33** | 21.44 | 0.38 |
| **SEQ ID NO:09** | 15.87 | 0.79 | **SEQ ID NO:34** | 20.44 | 0.75 |
| **SEQ ID NO:10** | 14.54 | 0.65 | **SEQ ID NO:35** | 21.58 | 1.91 |
| **SEQ ID NO:11** | 15.19 | 0.64 | **SEQ ID NO:36** | 23.14 | 1.52 |
| **SEQ ID NO:12** | 18.39 | 1.10 | **SEQ ID NO:37** | 22.41 | 1.64 |
| **SEQ ID NO:13** | 16.56 | 1.11 | **SEQ ID NO:38** | 20.22 | 1.47 |
| **SEQ ID NO:14** | 27.88 | 2.68 | **SEQ ID NO:39** | 22.29 | 1.23 |
| **SEQ ID NO:15** | 30.55 | 2.03 | **SEQ ID NO:40** | 37.52 | 2.81 |
| **SEQ ID NO:16** | 24.87 | 1.08 | **SEQ ID NO:41** | 28.65 | 3.14 |
| **SEQ ID NO:17** | 29.16 | 1.50 | **SEQ ID NO:42** | 25.68 | 1.51 |
| **SEQ ID NO:18** | 18.79 | 1.41 | **SEQ ID NO:43** | 52.02 | 2.81 |
| **SEQ ID NO:19** | 16.86 | 1.15 | **SEQ ID NO:44** | 52.92 | 2.01 |
| **SEQ ID NO:20** | 26.03 | 1.03 | **SEQ ID NO:45** | 46.01 | 2.62 |
| **SEQ ID NO:21** | 15.63 | 1.84 | **SEQ ID NO:46** | 56.10 | 0.95 |
| **SEQ ID NO:22** | 40.90 | 1.22 | **SEQ ID NO:47** | 19.10 | 2.01 |
| **SEQ ID NO:23** | 22.82 | 1.06 | **SEQ ID NO:48** | 29.67 | 2.19 |
| **SEQ ID NO:24** | 32.59 | 1.39 | **SEQ ID NO:49** | 30.23 | 1.86 |
| **SEQ ID NO:25** | 33.02 | 1.58 | **SEQ ID NO:50** | 23.37 | 1.69 |

**Table 2-2. IC₅₀ values of polypeptides for blocking CD36 binding to TSP-1 protein**

| **Sequence NO.** | **IC50(µM)** | **Sequence NO.** | **IC50 (µM)** |
|---|---|---|---|
| **SEQ ID NO:06** | 12.68 | **SEQ ID NO:36** | 4.85 |
| **SEQ ID NO:14** | 4.76 | **SEQ ID NO:40** | 7.70 |
| **SEQ ID NO:15** | 5.65 | **SEQ ID NO:43** | 3.62 |
| **SEQ ID NO:22** | 7.20 | **SEQ ID NO:44** | 6.71 |
| **SEQ ID NO:23** | 22.89 | **SEQ ID NO:45** | 5.96 |
| **SEQ ID NO:24** | 10.61 | **SEQ ID NO:46** | 1.40 |
| **SEQ ID NO:25** | 7.40 | **SEQ ID NO:48** | 11.71 |
| **SEQ ID NO:26** | 3.40 | **SEQ ID NO:49** | 10.12 |
| **SEQ ID NO:33** | 14.34 | **SEQ ID NO:50** | 10.60 |

### Example 3. Inhibition Assay of polypeptides on TSP-1-dependent TGF-β activation in a cell-based model

In this Example, the inhibitory effects of the polypeptides on TSP-1-dependent TGF-β activation were detected using a model based on a high TSP-1 expressing cell. In the assay, THP-1 cells (American Typical Culture Collection, ATCC, TIB-20) were induced by phorbol ester (Phorbol 12-myristate 13-acetate, PMA) (Sigma, Catalog No. P1585) and differentiated into macrophage-like cells with distinct macrophage expression profiles. In the cell-based model, TSP-1 protein expression was significantly increased and L-TGF-β was secreted; and in the absence of factors interfering with L-TGF-β activation, cell-secreted fibrinolytic enzyme would recognize and cleave the TSP-1/L-TGF-β complex bound to CD36 receptor on cell-surface, resulting in the production of increased amounts of active TGF-β.

The experiment was performed as follows: THP-1 cells were inoculated in a 96-well plate, and treated with 300 nM PMA for differentiation and adherence to form macrophage-like cells. 100 µM polypeptide was added (For the negative control group, an equal volume of PBS (the solvent for the polypeptide) was added, while the positive control group received an equal concentration of the polypeptide shown in SEQ ID NO: 50.) The cells were treated for 24 hours, and then a 293 reporter cell strain, 293-TGFβ Res cell strain (novoprotein, Catalog No. XCC03-1), was added for detecting TGF-β activity, and co-cultivated for 24 hours. Bio-Lite Luciferase Assay lysate comprising fluorescent substrate (Vazyme, Catalog No. DD1201-01) was added, and the fluorescent intensity was detected using a multifunctional microplate reader (TECAN, model Infinite 200 Pro). The inhibitory effect of the polypeptide on TGF-β activation was assessed by calculating the ratio of the fluorescence signal from the polypeptide-treated groups to that of the negative control group. The experimental results were shown in Table 3. In this cell-based model, the polypeptides exhibited varying degrees of inhibition on TGF-β activation, indicating that the polypeptides of the present invention inhibit TSP-1-dependent TGF-β activation.

**Table 3. Inhibition of TSP-1-dependent TGF-β activation by polypeptides in cell-based model**

| **SEQ ID NO:** | **Mean (relative fluorescence intensity)** | **±SD** |
|---|---|---|
| **Negative control group** | 1.00 | / |
| **Positive control group** | 0.84 | 0.10 |
| **SEQ ID NO:03** | 0.70 | 0.07 |
| **SEQ ID NO:04** | 0.77 | 0.05 |
| **SEQ ID NO:05** | 0.83 | 0.14 |
| **SEQ ID NO:06** | 0.65 | 0.16 |
| **SEQ ID NO:14** | 0.70 | 0.03 |
| **SEQ ID NO:17** | 0.78 | 0.08 |
| **SEQ ID NO:22** | 0.74 | 0.00 |
| **SEQ ID NO:24** | 0.57 | 0.11 |
| **SEQ ID NO:25** | 0.73 | 0.13 |
| **SEQ ID NO:26** | 0.68 | 0.03 |
| **SEQ ID NO:31** | 0.79 | 0.10 |
| **SEQ ID NO:33** | 0.67 | 0.02 |
| **SEQ ID NO:36** | 0.83 | 0.06 |
| **SEQ ID NO:40** | 0.77 | 0.11 |
| **SEQ ID NO:41** | 0.62 | 0.04 |
| **SEQ ID NO:42** | 0.67 | 0.03 |
| **SEQ ID NO:43** | 0.65 | 0.11 |
| **SEQ ID NO:44** | 0.43 | 0.09 |
| **SEQ ID NO:45** | 0.52 | 0.11 |
| **SEQ ID NO:46** | 0.69 | 0.07 |
| **SEQ ID NO:47** | 0.80 | 0.19 |

### Example 4. Inhibition assay of polypeptides on active TGFβ level in a mouse model of fibrotic disease

This Example was to evaluate the inhibitory effect of polypeptides on active TGF-β level during the progression of fibrotic disease. In this Example, a mouse lung fibrosis model induced by bleomycin (BLM) was used. The BLM animal model offers advantages, including a close mimicry of the pathological features of the primary disease, ease of use, and good reproducibility. It is the most representative model for studying the mechanisms of fibrosis and is recommended for evaluating drug efficacy.

In this Example, 6 to 8-week-old male C57/6J mice weighing 18-20 g were used as subjects for the study. The experimental animals were randomized into groups, 8 animals in each group, and the expressions of pro-fibrotic factors were detected at the endpoint of the experiment (Day 8) in this model. In this example, an aerosol intratracheal metered dosing applicator (Yuyan, model YAN30012) was utilized for intratracheal administration during the modeling and drug delivery processes. The dosing was performed as follows (An Official American Thoracic Society Workshop Report: Use of Animal Models for the Preclinical Assessment of Potential Therapies for Pulmonary Fibrosis, Am J Respir Cell Mol Biol Vol 56, Iss 5, pp 667-679, May 2017): The experimental animals were anesthetized with aerosolized isoflurane (RWD, lot no. 22041701) using a small animal anesthesia machine (RWD, model R500IE). Animals in the appropriate anesthetic state were positioned supine with heads facing up and bodies tilted on an operating platform (tilted at approximately 60°), secured by their incisors. The practitioner gently lifted the tongue with tissue forceps in the right hand, pushing it outward and upward to open the animal's lower jaw. Holding the laryngoscope (Yuyan, model SR310-RW) in the left hand, the practitioner slid its anterior leaf-shaped blade along the midline of the tongue into the oral cavity until reaching the glottis, gently pressing the body and root of the tongue upward. The needle of the drug applicator was then inserted through the mouth into the glottis alongside the laryngoscope, and 50 µL of liquid was quickly injected. On the day of modeling (Day 1), the experimental animals were randomly assigned to groups and administered a 0.7 USP/kg dose of bleomycin solution (HanHui Pharmaceutical, Batch No. 20080511) via the tracheal route to induce the model. On Day 7, each peptide-treated group received a single intratracheal dose of 50 µL peptide drug (at a dosage of 3 mg/kg), while the model group was given an equal volume of solvent (saline, Kelong, Batch No. L221062501) as a control. Twenty-four hours after drug administration, the animals were dissected to collect lung tissue, which was ground in liquid nitrogen to extract total protein from the lung tissue. The active TGF-β content in the lung tissue of mice from each group was measured using the TGF-β ELISA kit (BOSTER, catalog number EK0515). The ratio of active TGF-β content between the peptide-treated group and the model group was calculated to evaluate the effect of the peptide on active TGF-β level. The results are presented in Table 4. In the fibrotic disease model, the peptide-treated groups exhibited varying degrees of reduction in active TGF-β level compared to the model group, indicating that the peptides of this invention have an inhibitory effect on the increase of active TGF-β level induced by lung injury during fibrosis progression.

**Table 4. Inhibition of active TGF-β level by polypeptides in a mouse fibrosis model**

| **SEQ ID NO:** | **Mean(%)** | **±SD(%)** |
|---|---|---|
| **BLM model group** | 100.00 | / |
| **SEQ ID NO:04** | 74.10 | 17.32 |
| **SEQ ID NO:05** | 74.15 | 9.22 |
| **SEQ ID NO:06** | 76.76 | 6.94 |
| **SEQ ID NO:07** | 71.66 | 13.70 |
| **SEQ ID NO:08** | 80.31 | 11.46 |
| **SEQ ID NO:09** | 72.26 | 16.09 |
| **SEQ ID NO:12** | 81.77 | 9.13 |
| **SEQ ID NO:14** | 92.49 | 20.22 |
| **SEQ ID NO:17** | 71.36 | 9.47 |
| **SEQ ID NO:22** | 82.72 | 11.59 |
| **SEQ ID NO:24** | 76.35 | 13.40 |
| **SEQ ID NO:25** | 86.63 | 12.31 |
| **SEQ ID NO:26** | 83.44 | 13.58 |
| **SEQ ID NO:27** | 89.89 | 14.07 |
| **SEQ ID NO:31** | 83.22 | 15.47 |
| **SEQ ID NO:33** | 86.61 | 12.53 |
| **SEQ ID NO:36** | 77.42 | 9.20 |
| **SEQ ID NO:40** | 68.27 | 11.95 |
| **SEQ ID NO:41** | 74.12 | 12.31 |
| **SEQ ID NO:42** | 80.59 | 14.06 |
| **SEQ ID NO:43** | 75.92 | 8.36 |
| **SEQ ID NO:44** | 81.73 | 8.92 |
| **SEQ ID NO:45** | 81.10 | 13.06 |
| **SEQ ID NO:46** | 79.36 | 7.53 |
| **SEQ ID NO:47** | 83.78 | 13.94 |
| **SEQ ID NO:48** | 82.97 | 13.37 |
| **SEQ ID NO:49** | 82.64 | 12.39 |

### Example 5. Inhibition assay of polypeptides on TGFβ downstream signaling pathway in a mouse model of fibrotic disease

This example aimed to evaluate the inhibitory effect of polypeptides on the TGF-β downstream signaling pathway during fibrosis progression. TGF-β intracellular signaling relies on the phosphorylation events of Smad family proteins. The increase in Smad2/3 phosphorylation levels is an important indicator that signal transduction has occurred in the TGF-β signaling pathway.

In this example, 6- to 8-week-old male C57/6J mice weighing 18-20 g were used as subjects for the study. The experimental animals were randomized into groups, eight animals in each group. In this example, intratracheal administration was performed using an aerosol intratracheal metered dosing applicator during both the modeling and drug delivery processes, as described in Example 4. On the day of modeling (Day 1), the experimental animals were randomly assigned to groups and administered a 0.7 USP/kg dose of bleomycin solution (HanHui Pharmaceutical, Batch No. 20080511) via the tracheal route to induce the model. On Day 7, each peptide-treated group received a single intratracheal dose of peptide drug at a dosage of 3 mg/kg, while the BLM model group was given an equal volume of solvent (saline) as a control. Twenty-four hours after drug administration, the animals were dissected, and the lung tissues were collected and frozen in liquid nitrogen for future use. Total protein from the lung tissue was extracted after grinding the tissue in liquid nitrogen, and the total P-Smad2/3 content in the tissue was measured by using P-Smad2/3 ELISA kit (CST, Catalog No.12001C). The ratio of the P-Smad2/3 content between the peptide-treated group and the model group was calculated to evaluate the effect of the peptide on TGFβ signaling. The results are presented in Table 5. In the fibrotic disease model, the peptide-treated groups exhibited varying degrees of reduction in P-Smad2/3 protein levels compared to the model group, indicating that the peptides of this invention have varying degrees of inhibitory effects on the signal transduction of TGFβ signaling pathway during fibrosis progression.

**Table 5. Inhibition of TGF-β signal transduction pathway by polypeptides in a mouse fibrosis model**

| **SEQ ID NO:** | **Mean(%)** | **SD(%)** |
|---|---|---|
| **BLM model group** | 100.00 | / |
| **SEQ ID NO:04** | 67.91 | 16.98 |
| **SEQ ID NO:05** | 72.35 | 13.03 |
| **SEQ ID NO:06** | 54.89 | 10.87 |
| **SEQ ID NO:07** | 80.71 | 9.93 |
| **SEQ ID NO:08** | 83.02 | 7.72 |
| **SEQ ID NO:09** | 89.41 | 3.96 |
| **SEQ ID NO:12** | 90.48 | 11.38 |
| **SEQ ID NO:14** | 87.39 | 12.95 |
| **SEQ ID NO:17** | 74.12 | 10.33 |
| **SEQ ID NO:22** | 55.32 | 17.35 |
| **SEQ ID NO:24** | 89.89 | 6.70 |
| **SEQ ID NO:25** | 65.07 | 11.80 |
| **SEQ ID NO:26** | 76.78 | 15.84 |
| **SEQ ID NO:27** | 88.69 | 12.87 |
| **SEQ ID NO:31** | 90.02 | 7.07 |
| **SEQ ID NO:33** | 71.09 | 6.64 |
| **SEQ ID NO:36** | 79.00 | 9.13 |
| **SEQ ID NO:40** | 83.20 | 12.84 |
| **SEQ ID NO:41** | 81.44 | 11.10 |
| **SEQ ID NO:42** | 85.16 | 9.85 |
| **SEQ ID NO:43** | 49.30 | 5.19 |
| **SEQ ID NO:44** | 79.93 | 12.84 |
| **SEQ ID NO:45** | 82.42 | 13.54 |
| **SEQ ID NO:46** | 74.60 | 10.54 |
| **SEQ ID NO:47** | 93.25 | 13.29 |
| **SEQ ID NO:48** | 80.57 | 18.86 |
| **SEQ ID NO:49** | 91.98 | 15.19 |

### Example 6. Inhibition assay of polypeptides on collagen deposition in a mouse model of fibrotic disease

This Example was to evaluate the inhibitory effect of polypeptides on collagen deposition in fibrotic tissues. In this example, 6- to 8-week-old male C57/6J mice weighing 18-20 g were used as subjects for the study. The experimental animals were randomized into groups, eight animals in each group. On the day of modeling (Day 1), a 0.7 USP/kg dose of bleomycin solution was administered via the tracheal route to induce the model. Treatment dosing started on Day 8. Each peptide-treated group received the peptide drug intratracheally at a dosage of 3 mg/kg, administered twice weekly for a total of four doses. The BLM model group received an equal volume of solvent (saline) as a control. The administration procedure followed that described in Example 4. Day 21 was set as the experimental endpoint for assessing the degree of fibrosis in the model.

On Day21, the animals were dissected to collect lung tissues, and after grinding the lung tissues in liquid nitrogen, hydroxyproline (HYP) was extracted from the tissue using acid hydrolysis. HYP is a unique amino acid found in collagen, and the determination of HYP content is an important indicator reflecting the metabolism of collagen tissue and the degree of fibrosis. 1 mL of 6N HCl was added per 100mg of tissue and thoroughly mixed, followed by hydrolysis at 121°C for 6 hours. The hydrolysate was centrifuged, and the resulting supernatant, along with a gradient of HYP standard solutions (Sigma, Catalog No.V900395-25G) at different concentrations, was used for HYP content determination. To each well of a 96-well plate, 10 µL of the hydrolysate supernatant was added and dried in an oven at 65°C. 100 µL per well of 1.4% chloramine T oxidizing solution (Sigma, Catalog No. 23270-50G) was added, and incubated at room temperature for 15 minutes. 100 µL per well of Ehrlich's color development solution (Solarbio, Catalog No. G1290) was added and incubated at 65°C for 30 minutes. The absorbance of each sample well was measured at 560 nm (OD560) using a multifunctional microplate reader (TECAN, model Infinite 200 Pro). The total HYP content in the lung tissue from each experimental group was calculated to determine the reduction percentage of HYP content in the peptide-treated group relative to the model group, and the inhibitory effect of the peptide molecule on collagen deposition at the fibrotic site was assessed. The results are presented in Table 6. In the fibrotic disease model, the peptide-treated groups exhibited varying degrees of reduction in HYP contents compared to the model group, indicating that the peptides of this invention have varying degrees of inhibitory effects on collagen deposition in fibrotic tissues.

**Table 6. Inhibition of polypeptides on collagen deposition in fibrotic tissues**

| **SEQ ID NO:** | **Mean (%)** | **±SD (%)** |
|---|---|---|
| **BLM model group** | 100.00 | / |
| **SEQ ID NO:06** | 79.51 | 15.71 |
| **SEQ ID NO:22** | 72.66 | 12.07 |
| **SEQ ID NO:25** | 77.75 | 6.94 |
| **SEQ ID NO:26** | 72.07 | 5.13 |
| **SEQ ID NO:33** | 82.23 | 7.66 |
| **SEQ ID NO:40** | 77.88 | 8.70 |
| **SEQ ID NO:43** | 72.18 | 13.76 |
| **SEQ ID NO:44** | 78.25 | 12.80 |
| **SEQ ID NO:46** | 80.16 | 13.34 |

### Example 7. Inhibition assay of polypeptides on inflammatory and fibrotic pathology in a mouse model of fibrotic disease

This Example was to assess the inhibitory effect of polypeptides on fibrotic pathology. 6- to 8-week-old male C57/6J mice weighing 18-20 g were used as subjects for the study. The experimental animals were randomized into groups, 6 animals in each group. On the day of modeling (Day 1), a single dose of 0.7 USP/kg of BLM solution was administered via the tracheal route to the BLM group and each peptide-treated group to induce the model. The negative control group received an equal volume of saline. Treatment dosing started on Day 8. Each peptide-treated group received the peptide drug intratracheally at a dosage of 3 mg/kg, while the positive control group received the peptide of SEQ ID NO: 50 at the same dosage, and the administration frequency was twice a week, with a total of 4 administrations. The BLM model group and the negative control group received an equal volume of solvent (saline) as a control. The procedure followed that described in Example 4.

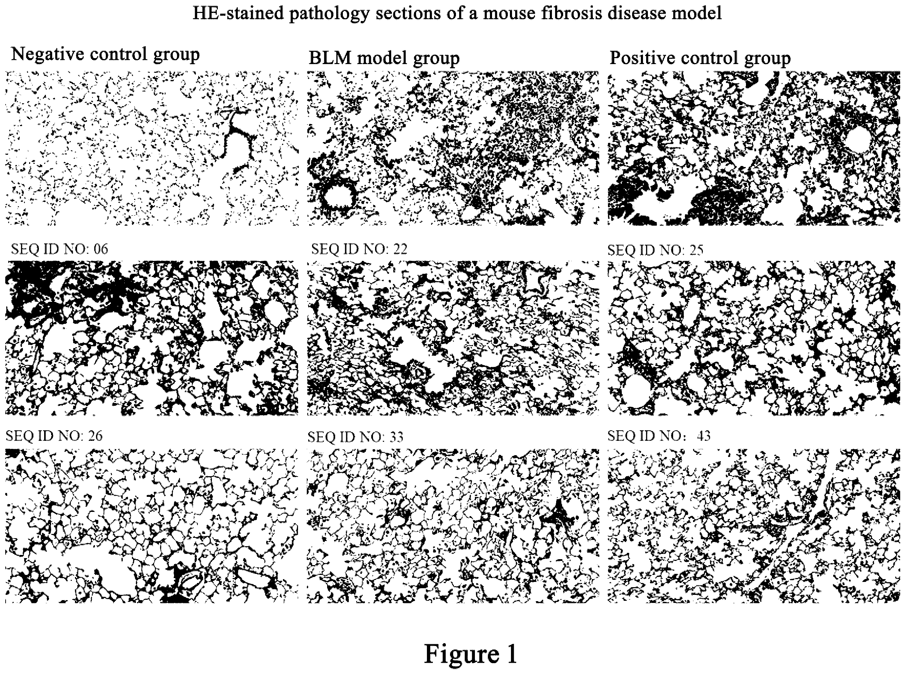

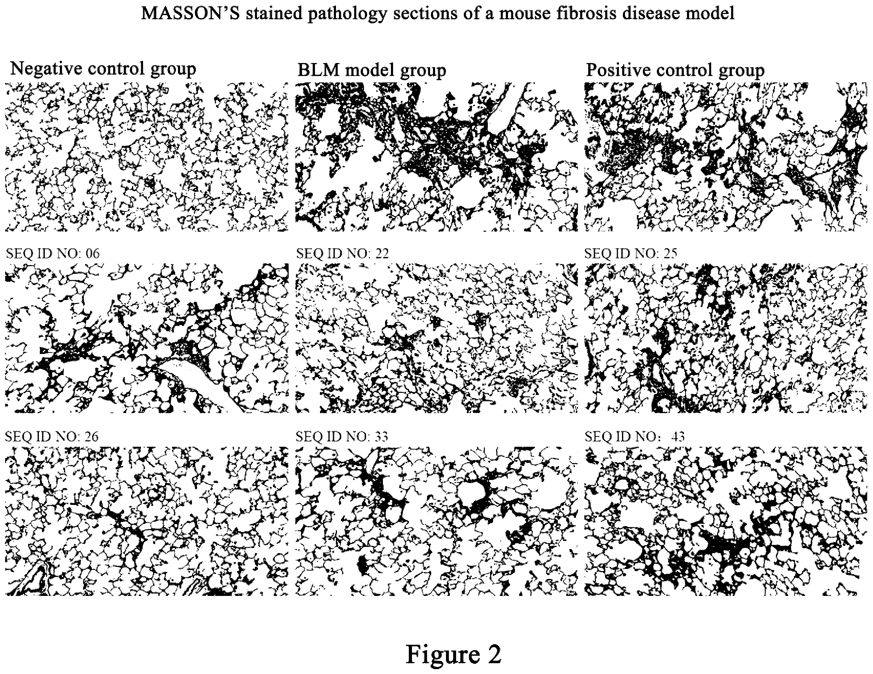

On Day 21, the lung tissues were dissected, washed with saline to remove blood stains, and dried with gauze. Histopathological analysis was then performed on the dissected lung tissues. The lung tissues were perfused with 0.5 mL of paraformaldehyde via the trachea and placed in 4 mL of paraformaldehyde. A small amount of gauze was added to ensure the lung tissues were completely immersed in the fixative solution. The tissues were fixed for more than 24 hours. One lobe of the left lung and four lobes of the right lung were trimmed to the maximum coronal plane, placed in an embedding cassette, dehydrated overnight, and then paraffin-embedded to make 3 µm sections. An autostainer (Leica, model ST5010) was used for Hematoxylin (Herst, lot no. 201905)-Eosin (Chron, lot no.2018070301) (HE) staining, and MASSON'S trichrome (Celestineblue, Chron, lot no. MKCH8129; Aniline blue, Yuanye, lot no. H70J11S115483; Ponceau, Sigma, lot no. SHBM2047) staining. After mounting the sections, they were scanned using a digital panoramic scanner (Zhiyue, model WS-10). For each lung lobe, ≤ 10 visual fields were captured under a 20x magnification. The severity of inflammation and the severity of fibrosis were assessed using HE staining and Masson's trichrome staining, respectively, to evaluate the inhibitory effects of the polypeptides on inflammatory infiltration and fibrotic deposition at pathological fibrotic sites. The results are presented in Figures 1 and 2.

Figure 1 shows HE-stained pathological sections (20x magnification). In the lung tissue of the negative control group, the alveolar cavity had a vacuolar-like and thin-walled structure. The BLM model group exhibited a significant infiltration of inflammatory cells and fibroblasts in the interstitium and alveoli, accompanied by marked thickening of alveolar interstitium and compression and deformation of alveolar structure. All peptide-treated groups exhibited varying degrees of reduction in inflammatory cells and decrease in the areas of the thickened alveolar septa.

Figure 2 shows the Masson's stained pathological sections (20x magnification). In the lung tissue of the negative control group mice, the collagen layer of the bronchial wall was relatively thin, and a small number of elongated, strip-like collagen fibres were observed distributed among the alveoli. In the BLM model group, the alveolar septa were widened, with significant deposition of collagen fibres, and fibrotic masses had formed in some areas. In each peptide-treated group, only a small amount of the alveolar septa was widened, and the area of collagen fibre deposition showed varying degrees of reduction compared to the BLM model group.

The results of the inflammatory and fibrotic scores are presented in Table 7. The inflammatory and fibrotic scores were significantly higher in the BLM model group compared to the negative control group. The inflammatory and fibrotic scores in each polypeptide administration group showed varying degrees of decrease compared to the model group.

**Table 7 Inhibition of polypeptides on inflammatory and fibrotic pathologies in a mouse fibrosis model**

| **SEQ ID NO:** | Inflammatory score | | Fibrosis score | |
|---|---|---|---|---|
| | **Mean** | **±SD** | **Mean** | **±SD** |
| **Negative control group** | 0.36 | 0.11 | 1.01 | 0.20 |
| **BLM model group** | 2.25 | 0.17 | 3.25 | 0.21 |
| **Positive control group** | 1.98 | 0.14 | 2.81 | 0.19 |
| **SEQ ID NO:06** | 1.65 | 0.29 | 2.65 | 0.16 |
| **SEQ ID NO:22** | 1.43 | 0.21 | 2.33 | 0.29 |
| **SEQ ID NO:25** | 1.39 | 0.18 | 2.47 | 0.34 |
| **SEQ ID NO:26** | 0.95 | 0.16 | 2.05 | 0.17 |
| **SEQ ID NO:33** | 1.27 | 0.25 | 2.64 | 0.28 |
| **SEQ ID NO:43** | 1.46 | 0.21 | 2.59 | 0.20 |

### Example 8. Inhibition assay of polypeptides on collagen deposition in a rat fibrotic disease model

This Example was to evaluate the inhibitory effect of polypeptides on collagen deposition at the pathological sites of pulmonary fibrosis. 6- to 8-week-old male SD rats weighing 200 to 220 g were used as subjects for the study. The experimental animals were randomized into groups, 8 animals in each group. On the day of modeling (Day 1), a single dose of 1.8 USP/kg of bleomycin solution was administered via the tracheal route to the model group and each peptide-treated group to induce the model. Starting on Day 1, each peptide-treated group received intratracheal administration of the peptide at a dosage of 2 mg/kg, with a dosing frequency of twice a week, for a total of four administrations. The model group received an equal volume of solvent (saline) as a control, with the volume being 100 µL. The procedure followed that described in Example 4. Day 15 was set as the experimental endpoint for assessing the degree of fibrosis in the model.

On Day 15, the animals were dissected to collect lung tissues. After grinding the rat lung tissues in liquid nitrogen, total hydroxyproline (HYP) was extracted from the tissues using acid hydrolysis, and the HYP content was determined by chloramine T oxidation colorimetry. 1 mL of 6N HCl was added per 100mg of tissue and thoroughly mixed, followed by hydrolysis at 121°C for 6 hours. The hydrolysate was centrifuged, and the resulting supernatant was used for HYP content determination. To a 96-well plate, the hydrolysate supernatant, or a gradient of HYP standard solutions (Sigma, Catalog No.V900395-25G) at different concentrations, was added in 10 µL per well, and dried in an oven at 65°C. 100 µL per well of 1.4% chloramine T oxidizing solution (Sigma, Catalog No. 23270-50G) was added, and incubated at room temperature for 15 minutes. 100 µL per well of Ehrlich's color development solution (Solarbio, Catalog No. G1290) was added and incubated at 65°C for 30 minutes. The absorbance of each sample well was measured at 560 nm (OD560) using a multifunctional microplate reader (TECAN, model Infinite 200 Pro). The total HYP content in the lung tissue from each experimental group was calculated to determine the reduction percentage of HYP content in the peptide-treated group relative to the model group, and the inhibitory effect of the peptide molecule on collagen deposition at the fibrotic site was assessed. The results are presented in Table 8. In the fibrotic disease model, the peptide-treated groups exhibited varying degrees of reduction of HYP contents compared to the model group, indicating that the peptides of this invention have varying degrees of inhibitory effects on collagen deposition in fibrotic tissues.

**Table 8. Inhibition of polypeptides on collagen deposition in fibrosis model**

| **SEQ ID NO:** | **Mean (%)** | **±SD (%)** |
|---|---|---|
| **Model group** | 100.00 | / |
| **SEQ ID NO:06** | 80.30 | 10.71 |
| **SEQ ID NO:22** | 68.30 | 14.13 |
| **SEQ ID NO:25** | 81.45 | 9.61 |
| **SEQ ID NO:26** | 78.75 | 10.42 |
| **SEQ ID NO:33** | 80.28 | 12.83 |
| **SEQ ID NO:40** | 72.93 | 12.05 |
| **SEQ ID NO:43** | 72.91 | 13.22 |
| **SEQ ID NO:44** | 65.55 | 11.45 |
| **SEQ ID NO:46** | 66.46 | 13.56 |

### Example 9. Inhibition assay of polypeptides on fibrotic pathology in a rat model of fibrotic disease

This Example was to assess the inhibitory effect of polypeptide administration on the expression levels of pro-fibrotic factors at pathological fibrotic sites. 6- to 8-week-old male SD rats weighing 200 to 220 g were used as subjects for the study. The experimental animals were randomized into groups, 6 animals in each group. On the day of modeling (Day 1), a single dose of 1.8 USP/kg of bleomycin solution was administered via the tracheal route to the BLM model group and each peptide-treated group to induce the model. The negative control group was given an equal volume of saline. Starting on Day 1, each peptide-treated group received intratracheal administration of the peptide at a dosage of 2 mg/kg, with a dosing frequency of twice a week, for a total of four administrations. The BLM model group and the negative control group received an equal volume of solvent (saline) as a control, with the volume being 100 µL. The procedure followed that described in Example 4.

On Day 15, the lung tissues were dissected, washed with saline to remove blood stains, dried with gauze, and weighed. The lung index was calculated (lung index = (wet lung weight/body weight) × 1000. This index reflects the general condition of the lung tissue, including edema, inflammation, and fibrosis). Histopathological analysis was performed on the dissected lung tissues. The lung tissues were perfused with 5 mL of paraformaldehyde via the trachea and placed in 40 mL of paraformaldehyde. A small amount of gauze was added to ensure the lung tissues were completely immersed in the fixative solution. The tissues were fixed for more than 24 hours. One lobe of the left lung and four lobes of the right lung were trimmed to the maximum coronal plane, and the left and right lungs were individually placed in two embedding cassettes, and then paraffin-embedded to make 3µm sections. Masson's trichrome staining was performed as described in Example 7. After digital scanning, for each lung lobe, ≤ 10 visual fields were captured under a 10x magnification. The severity score of fibrosis was assessed to evaluate the inhibitory effects of the polypeptides on fibrotic deposition at pathological fibrotic sites.

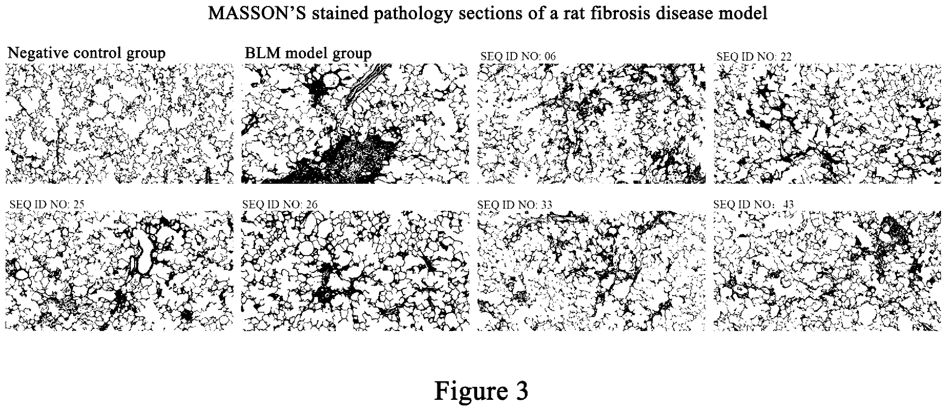

The results are shown in Figure 3 (10x magnification). In the negative control group, the collagen layer of the bronchial wall in the rat lung tissues was relatively thin, and a small number of elongated, strip-like collagen fibres were observed distributed among the alveoli. In the BLM model group, the alveolar septa in the lung tissues were widened, with significant deposition of collagen fibres, and fibrotic masses had formed in some areas. In each peptide-treated group, a small number of alveolar septa were widened, and the area of collagen fibre deposition showed varying degrees of reduction compared to the BLM model group.

The results of the fibrotic scores are presented in Table 9. The lung index and fibrotic severity score in the BLM model group were significantly increased compared to the negative control group. The lung index and fibrotic severity score in each peptide-treated group showed varying degrees of decrease compared to the model group.

**Table 9 Inhibition of polypeptides on fibrosis pathologies in a rat fibrosis model**

| **SEQ ID NO:** | **Lung index** | | **Fibrosis score** | |
|---|---|---|---|---|
| | **Mean(‰)** | **±SD** | **Mean** | **±SD** |
| **Negative control group** | 4.62 | 0.12 | 0.96 | 0.23 |
| **BLM model group** | 8.63 | 0.72 | 3.34 | 0.18 |
| **SEQ ID NO:06** | 7.17 | 0.32 | 2.66 | 0.31 |
| **SEQ ID NO:22** | 7.39 | 0.37 | 2.70 | 0.13 |
| **SEQ ID NO:25** | 7.42 | 0.29 | 2.62 | 0.23 |
| **SEQ ID NO:26** | 7.17 | 0.42 | 2.43 | 0.22 |
| **SEQ ID NO:33** | 7.37 | 0.34 | 2.63 | 0.21 |
| **SEQ ID NO:43** | 7.11 | 0.67 | 2.72 | 0.29 |

### Example 10. Inhibition assay of polypeptides on tumor cell migration ability

TGF-β plays a dual role in the tumor microenvironment (TME). In early tumor stages, the TGF-β signaling pathway induces apoptosis and suppresses tumor cell proliferation. In advanced tumor stages, TGF-β functions as an immunosuppressive cytokine, inhibiting the immune response and promoting tumor progression by regulating tumor cell motility and metastasis, and the like.

This Example was to assess the effects of polypeptides on the migration abilities of different tumor cells. The experiment was performed using transwell nested chambers (membrane pore size of 8 µm), where THP-1 cells were inoculated in the lower chamber and treated with 300 nM PMA (Sigma, Catalog No.P1585) for differentiation and chamber-wall adherence, and then treated with 100 µM polypeptide (in the negative control, an equal volume of solvent was added) for 48 hours. The upper chamber was inoculated with tumor cells, human lung adenocarcinoma A549 cells (American Typical Culture Collection, ATCC, CCL-18), and co-cultured for 24 hours; or inoculated with human liver cancer HepG2/C3A cells (ATCC, CRL-10741), and co-cultured for 24 h; or inoculated with human breast cancer MCF7 cells (ATCC, HTB-22), and co-cultured for 48 h. After that, the upper chamber was taken out, fixed with 70% ethanol for 10 min and washed, then stained with Giemsa staining solution (Beyotime, Catalog No.C0131) for 45 min. After washing with double distilled water 2-3 times, unmigrated cells were wiped off the upper layer with a cotton swab. After the chamber was dried, it was observed under a microscope, and five different fields of view under a 100x magnification were randomly selected for counting and statistics. The results are presented in Table 10. The polypeptides had significant inhibitory effects on the migratory abilities of human lung adenocarcinoma cells, human liver cancer cells, and human breast cancer cells.

**Table 10. Inhibitory effects of polypeptides on the migration abilities of tumor cells**

| **SEQ ID NO:** | **A549** | | **HEPG2/C3A** | | **MCF7** | |
|---|---|---|---|---|---|---|
| | **Mean (%)** | **±SD** | **Mean (%)** | **±SD** | **Mean (%)** | **±SD** |
| **Negative control group** | 100.00 | / | 100.00 | / | 100.00 | / |
| **SEQ ID NO:04** | 57.90 | 8.66 | 81.13 | 1.96 | 62.78 | 6.50 |
| **SEQ ID NO:05** | 63.92 | 4.26 | 74.72 | 4.40 | 42.55 | 5.83 |
| **SEQ ID NO:06** | 80.41 | 9.68 | 81.77 | 5.31 | 51.95 | 4.48 |
| **SEQ ID NO:22** | 59.45 | 5.64 | 76.69 | 7.21 | 48.64 | 3.74 |
| **SEQ ID NO:25** | 62.95 | 8.52 | 63.99 | 8.53 | 61.75 | 4.01 |
| **SEQ ID NO:26** | 73.25 | 11.43 | 55.16 | 10.34 | 63.23 | 2.73 |
| **SEQ ID NO:33** | 62.89 | 5.26 | 72.07 | 5.54 | 47.13 | 5.81 |
| **SEQ ID NO:40** | 67.97 | 7.77 | 67.27 | 7.00 | 51.27 | 5.94 |
| **SEQ ID NO:43** | 83.37 | 11.25 | 71.02 | 10.96 | 73.84 | 8.99 |
| **SEQ ID NO:44** | 73.63 | 6.29 | 70.63 | 14.08 | 68.24 | 5.19 |
| **SEQ ID NO:46** | 36.12 | 2.52 | 64.77 | 9.45 | 57.98 | 4.81 |

### Example 11. Inhibition of polypeptides on ECM-related gene expression in a cell-based model of liver fibrosis

This Example was to evaluate the inhibitory effect of polypeptides on the expression of extracellular matrix (ECM)-related genes in a cellular model of liver fibrosis. The experiment utilized human hepatic stellate cells (LX-2), which are the primary source of myofibroblasts in the liver. Upon activation, hepatic stellate cells transform into myofibroblasts, which secrete ECM in live and play a direct role in the development of hepatic fibrosis. Transient stimulation of LX-2 cells with TGF-β1 can initiate a positive feedback regulation of TSP-1-TGF-β1 activation, which continuously enhances myofibroblast characteristics, including ECM production.

This experiment was performed as follows. LX-2 cells (Procell, CL-0560) were stimulated by 20 ng/mL TGF-β1 (Genscript, Catalog No.Z03411) for 24 h, then added 100 µM polypeptide and cultured for 48 h. An equal volume of solvent was added to the model group, and LX-2 cells without any treatment were used as a blank control. After that, LX-2 cells were collected and total RNA was extracted for RT-qPCR to detect the expression of ECM-related genes, EDA-Fibronectin (fibronectin, FN) and Collagen I (COL1). The assay results are presented in Table 11. The polypeptides were able to reduce the expressions of FN and COLI genes in LX-2 and inhibit ECM production.

**Table 11. Inhibitory effects of polypeptides on the expression of ECM-related genes in cellular models of liver fibrosis**

| **SEQ ID NO:** | **COL I** | | **FN** | |
|---|---|---|---|---|
| | **Mean** | **±SD** | **Mean** | **±SD** |
| **Blank control group** | 1.00 | / | 1.00 | / |
| **Model group** | 6.62 | 0.33 | 11.38 | 0.12 |
| **SEQ ID NO:06** | 4.57 | 0.48 | 5.72 | 0.27 |
| **SEQ ID NO:22** | 4.63 | 0.60 | 6.62 | 0.33 |
| **SEQ ID NO:25** | 4.00 | 0.39 | 6.47 | 0.64 |
| **SEQ ID NO:26** | 4.30 | 0.27 | 5.79 | 0.88 |
| **SEQ ID NO:33** | 4.49 | 0.33 | 6.80 | 0.87 |
| **SEQ ID NO:43** | 4.63 | 0.34 | 5.80 | 0.34 |

### Overview of Sequence Listing

The present application is accompanied with a sequence listing. The following table provides information about each peptide shown in SEQ ID NO in the Sequence Listing, wherein Ac denotes acetyl group and NH₂ denotes amino group.

| **SEQ ID NO:** | **Peptide shown in SEQ ID NO** |
|---|---|
| SEQ ID NO:01 | Ac-Gln-Asp-Pro-Glu-Asp-NH₂ |
| SEQ ID NO:02 | Ac-Gln-Asp-Ala-Glu-Asp-NH₂ |
| SEQ ID NO:03 | Ac-Thr-Gln-Asp-Pro-Glu-Asp-NH₂ |
| SEQ ID NO:04 | Ac-Thr-Gln-Asp-Ala-Glu-Asp-NH₂ |
| SEQ ID NO:05 | Ac-Gln-Asp-Ala-Glu-Asp-Asn-NH₂ |
| SEQ ID NO:06 | Ac-Thr-Gln-Asp-Ala-Glu-Asp-Asn-NH₂ |
| SEQ ID NO:07 | Ac-Ser-Gln-Asp-Ala-Glu-Asp-Asn-NH₂ |
| SEQ ID NO:08 | Ac-Thr-Asn-Asp-Ala-Glu-Asp-Asn-NH₂ |
| SEQ ID NO:09 | Ac-Thr-Glu-Asp-Ala-Glu-Asp-Asn-NH₂ |
| SEQ ID NO:10 | Ac-Thr-Ser-Asp-Ala-Glu-Asp-Asn-NH₂ |
| SEQ ID NO:11 | Ac-Thr-Gln-Asp-Ser-Glu-Asp-Asn-NH₂ |
| SEQ ID NO:12 | Ac-Thr-Gln-Asp-Ala-Glu-Asp-Leu-NH₂ |
| SEQ ID NO:13 | Ac-Thr-Gln-Asp-Ala-Glu-Asp-Pro-NH₂ |
| SEQ ID NO:14 | Ac-Thr-Gln-Asp-Pro-Glu-Asp-Asn-NH₂ |
| SEQ ID NO:15 | Ac-Thr-Gln-Asp-Ala-Glu-Asp-Asn-Thr-NH₂ |
| SEQ ID NO:16 | Ac-Gln-Asp-Ala-Glu-Asp-Asn-Thr-Ala-NH₂ |
| SEQ ID NO:17 | Ac-Thr-Gln-Asp-Ala-Glu-Asp-Asn-Thr-Val-NH₂ |
| SEQ ID NO:18 | Ac-Ser-Gln-Asp-Ala-Glu-Asp-Asn-Thr-Val-NH₂ |
| SEQ ID NO:19 | Ac-Thr-Asn-Asp-Ala-Glu-Asp-Asn-Thr-Val-NH₂ |
| SEQ ID NO:20 | Ac-Thr-Gln-Asp-Pro-Glu-Asp-Asn-Thr-Val-NH₂ |
| SEQ ID NO:21 | Ac-Thr-Gln-Asp-Ser-Glu-Asp-Asn-Thr-Val-NH₂ |
| SEQ ID NO:22 | Ac-Thr-Gln-Asp-Ala-Glu-Asp-Pro-Thr-Ala-NH₂ |
| SEQ ID NO:23 | Ac-Gln-Asp-Ala-Glu-Asp-Asn-Thr-Ala-Ser-NH₂ |
| SEQ ID NO:24 | Ac-Thr-Gln-Asp-Ala-Glu-Asp-Asn-Thr-Ala-Ser-NH₂ |
| SEQ ID NO:25 | Ac-Thr-Gln-Asp-Ala-Glu-Asp-Pro-Thr-Ala-Ser-NH₂ |
| SEQ ID NO:26 | Ac-Thr-Gln-Asp-Pro-Glu-Asp-Asn-Thr-Ala-Ser-NH₂ |
| SEQ ID NO:27 | Ac-Thr-Gln-Asp-Ala-Glu-Asp-Asn-Thr-Val-Ser-Phe-NH₂ |
| SEQ ID NO:28 | Ac-Ser-Gln-Asp-Ala-Glu-Asp-Asn-Thr-Val-Ser-Phe-NH₂ |
| SEQ ID NO:29 | Ac-Thr-Glu-Asp-Ala-Glu-Asp-Asn-Thr-Val-Ser-Phe-NH₂ |
| SEQ ID NO:30 | Ac-Thr-Gln-Asp-Pro-Glu-Asp-Asn-Thr-Val-Ser-Phe-NH₂ |
| SEQ ID NO:31 | Ac-Thr-Gln-Asp-Ala-Glu-Asp-Asn-Thr-Ala-Ser-Phe-NH₂ |
| SEQ ID NO:32 | Ac-Thr-Gln-Asp-Ala-Glu-Asp-Asn-Thr-Ala-Ser-Tyr-NH₂ |
| SEQ ID NO:33 | Ac-Thr-Gln-Asp-Ala-Glu-Asp-Asn-Thr-Ala-Ser-His-NH₂ |
| SEQ ID NO:34 | Ac-Thr-Gln-Asp-Ala-Glu-Asp-Asn-Thr-Ala-Ser-Trp-NH₂ |
| SEQ ID NO:35 | Ac-Thr-Gln-Asp-Ala-Glu-Asp-Asn-Thr-Ala-Ser-His-Leu-NH₂ |
| SEQ ID NO:36 | Ac-Thr-Gln-Asp-Ala-Glu-Asp-Asn-Thr-Val-Ser-Phe-Leu-Gln-NH₂ |
| SEQ ID NO:37 | Ac-Ser-Gln-Asp-Ala-Glu-Asp-Asn-Thr-Val-Ser-Phe-Leu-Gln-NH₂ |
| SEQ ID NO:38 | Ac-Thr-Ser-Asp-Ala-Glu-Asp-Asn-Thr-Val-Ser-Phe-Leu-Gln-NH₂ |
| SEQ ID NO:39 | Ac-Thr-Gln-Asp-Pro-Glu-Asp-Asn-Thr-Val-Ser-Phe-Leu-Gln-NH₂ |
| SEQ ID NO:40 | cyclo[Thr-Gln-Asp-Ala-Glu-Asp-Asn], wherein the backbone is head to tail cyclized. |
| SEQ ID NO:41 | cyclo[Thr-Gln-Asp-Pro-Glu-Asp-Asn], wherein the backbone is head to tail cyclized. |
| SEQ ID NO:42 | Thr-Gln-cyclo[Asp-Ala-Glu-Asp-Asn-Lys], wherein the side chains of the residue Asp at position 3 and the residue Lys at position 8 form an amide bridge, resulting in cyclization. |
| SEQ ID NO:43 | Ac-Thr-cyclo[Cys-Asp-Ala-Glu-Asp-Asn-Cys], wherein the side chains of the residue Cys at position 2 and the residue Cys at position 8 form a disulfide, resulting in cyclization. |
| SEQ ID NO:44 | Ac-Thr-cyclo[Cys-Asp-Ala-Glu-Asp-Asn-Cys]-NH₂, wherein the side chains of the residue Cys at position 2 and the residue Cys at position 8 form a disulfide bond and cyclized. |
| SEQ ID NO:45 | cyclo[Gln-Asp-Ala-Glu-Asp-Asn-Thr], wherein the backbone is head to tail cyclized |
| SEQ ID NO:46 | cyclo[Thr-Gln-Asp-Pro-Glu-Asp-Asn-Thr-Ala-Ser], wherein the backbone is head to tail cyclized |
| SEQ ID NO:47 | Ac-Thr-Gln-Asp-Pro-Ala-Glu-Asp-NH₂ |
| SEQ ID NO:48 | Ac-Thr-Gln-Asp-Pro-Ala-Glu-Asp-Asn-NH₂ |
| SEQ ID NO:49 | Ac-Thr-Gln-Asp-Ala-Glu-Asp-Asn-Thr-Gln-Asp-NH₂ |
| SEQ ID NO:50 | Tyr-Arg-Val-Arg-Phe-Leu-Ala-Lys-Glu-Asn-Val-Thr-Gln-Asp-Ala-Glu-Asp-Asn |
| SEQ ID NO: 51 | Thr-Gln-Asp-Ala-Glu-Asp-Asn-Thr-Val-Ser-Phe-Leu-Gln |

## Claims

1. A peptide having an amino acid sequence of formula (I) or a pharmaceutically acceptable salt, solvate, or prodrug thereof,
R¹-X¹-X²- X³-X⁴- X⁵- X⁶-X⁷-R² (I),
wherein,
R¹ is acetyl or absent;
R² is amino or absent;
X¹ is absent or selected from T and S;
X² is selected from naturally occurring amino acids or non-naturally occurring amino acids, preferably selected from polar side-chain amino acids, such as uncharged polar side-chain amino acids S, C, G, N, Q, T, Y, or negatively charged polar side-chain amino acids D and E, and more preferably selected from amino acids N, C, Q, S, T, E and D; and more preferably selected from amino acids Q, C, N, E and S;
X³ is D;
X⁴ is selected from small side-chain amino acids, such as A, C, G, P, S, T and V, more preferably selected from amino acids A, P and S, more preferably selected from amino acids A and P;
X⁵ is E;
X⁶ is D;
X⁷ is either absent or a sequence of 1, 2, 3, 4, 5, 6, or 7 amino acids, selected sequentially from the amino acid sequence of Z¹Z²Z³SZ⁴LQ, starting from the amino terminal to the carboxyl terminal,
wherein Z¹ is an amino acid of N, Q, P, A, L, V, M, or I, preferably N, P, or L, most preferably N or P;
Z² is an amino acid of T, S, V, C, A, K, or R, preferably T, C, or K, more preferably, if Z² is an amino acid of C or K, then Z² forms a covalent linkage with the side chain of the amino acid X² or X³,
Z³ is an amino acid of A, V, L, or I, preferably A or V,
Z⁴ is an amino acid of F, Y, H, or W, preferably F or H;
optionally, if X⁴ is A, then 0 to 1 small side-chain amino acid, such as P or G, preferably P, is inserted between X³ and X⁴;
optionally, if X⁷ consists of 1 to 5 amino acids, then the peptide has 0 to 2 additional amino acid residues added after X⁷, such as 1 to 2 amino acid residues selected from amino acid residues of Q, E, D and N added, or 1 amino acid residue selected from C or K added.

2. The peptide or the pharmaceutically acceptable salt, solvate or prodrug thereof according to claim 1, wherein,
X¹ is absent or selected from T and S;
X² is selected from amino acids Q, N, C, S, T, E and D;
X³ is D;
X⁴ is selected from A, C, G, P, S, T and V;
X⁵ is E;
X⁶ is D;
X⁷ is either absent or a sequence of 1, 2, 3, 4, 5, 6, or 7 amino acids, selected sequentially from the amino acid sequence of Z¹Z²Z³SZ⁴LQ, starting from the amino terminal to the carboxyl terminal, wherein,
Z¹ is N, Q, P, A, L, V, M, or I;
Z² is T, K or C;
Z³ is A or V;
Z⁴ is F, Y, H, or W.

3. The peptide or the pharmaceutically acceptable salt, solvate or prodrug thereof according to any one of claims 1-2, wherein.
X¹ is selected from T and S;
X² is selected from Q, N, E, S, and C;
X³ is D;
X⁴ is selected from A and P;
X⁵ is E;
X⁶ is D.

4. The peptide or the pharmaceutically acceptable salt, solvate or prodrug thereof according to any one of claims 1-3, wherein the peptide comprises an amino acid sequence selected from:
- DAED;
- DPED;
- DAEDN;
- DPEDN; or
- DAEDP.

5. The peptide or the pharmaceutically acceptable salt, solvate or prodrug thereof according to any one of claims 1-4, wherein X⁷ consists of Z¹, Z¹Z², Z¹Z²Z³, Z¹Z²Z³S, Z¹Z²Z³SZ⁴, Z¹Z²Z³SZ⁴L, or Z¹Z²Z³SZ⁴LQ, and preferably, X⁷ consists of Z¹Z²Z³, Z¹Z²Z³S or Z¹Z²Z³SZ⁴, and wherein
Z¹ is N, P or L;
Z² is T, K or C;
Z³ is A or V;
Z⁴ is F, Y, H, or W.

6. The peptide or the pharmaceutically acceptable salt, solvate or prodrug thereof according to claim 5, wherein.
Z¹ is N or P;
Z² is T, K or C;
Z³ is A or V, preferably A;
Z⁴ is F or H.

7. The peptide or the pharmaceutically acceptable salt, solvate or prodrug thereof according to any one of claims 1-6, wherein X⁷ is an amino acid sequence selected from: N; P; L; NTA; NTV; PTA; NTAS; PTAS; NTVSF; NTASF; NTASH; and NTVSFLQ.

8. The peptide or the pharmaceutically acceptable salt, solvate or prodrug thereof according to any one of claims 1-7, wherein X¹ is T.

9. The peptide or the pharmaceutically acceptable salt, solvate or prodrug thereof according to any one of claims 1-8, wherein X⁴ is A.

10. The peptide or the pharmaceutically acceptable salt, solvate or prodrug thereof according to any one of claims 1-8, wherein X⁴ is P.

11. The peptide or the pharmaceutically acceptable salt, solvate or prodrug thereof according to any one of claims 1-10, wherein Z¹ is N.

12. The peptide or the pharmaceutically acceptable salt, solvate or prodrug thereof according to any one of claims 1-10, wherein Z¹ is P.

13. The peptide or the pharmaceutically acceptable salt, solvate or prodrug thereof according to any one of claims 1-12, wherein Z³ is A.

14. The peptide or the pharmaceutically acceptable salt, solvate or prodrug thereof according to any one of claims 1-13, wherein X² is Q.

15. The peptide or the pharmaceutically acceptable salt, solvate or prodrug thereof according to any one of claims 1-14, wherein Z² is T.

16. The peptide or the pharmaceutically acceptable salt, solvate, or prodrug thereof according to any one of claims 1-13, wherein X² is C, Z² is C, and the side chains of amino acids X² and Z² are covalently linked to form a disulfide.

17. The peptide or the pharmaceutically acceptable salt, solvate, or prodrug thereof according to any one of claims 1-13, wherein X³ is D, Z² is K, and the side chains of amino acids X³ and Z² are covalently linked to form a lactam bond.

18. The peptide or the pharmaceutically acceptable salt, solvate or prodrug thereof according to any one of claims 1-17, wherein R¹ is acetyl.

19. The peptide or the pharmaceutically acceptable salt, solvate or prodrug thereof according to any one of claims 1-18, wherein R² is amino.

20. The peptide or the pharmaceutically acceptable salt, solvate or prodrug thereof according to any one of claims 1-19, wherein the peptide consists of 5 to 13 amino acids, preferably consists of 7, 8, 9, 10, or 11 amino acids.

21. The peptide or the pharmaceutically acceptable salt, solvate or prodrug thereof according to any one of claims 1-20, wherein the peptide is a linear peptide or a cyclic peptide.

22. The peptide or the pharmaceutically acceptable salt, solvate or prodrug thereof according to any one of claims 1-21, wherein the peptide is a cyclic peptide, preferably the cyclic peptide is in head-to-tail cyclization, in D-K side-chain cyclization, or in disulfide cyclization,
wherein the cyclized region of the peptide comprises the core motif (DXED), preferably, the peptide is cyclized through the coupling of the side chains of amino acids at positions 3 and 8; or is cyclized through the coupling of the side chains of amino acids at positions 2 and 8, or
preferably, wherein X³ is D, Z² is K, and the peptide is cyclized through a lactam bond formed between the side chains of X³ and Z²; or
preferably, wherein X² is C, Z² is C, and wherein the peptide is cyclized through a disulfide bond formed between the side chains of X² and Z²;
more preferably, if Z² is an amino acid of C or K, then X⁷ consists of Z¹Z² and X¹ is T or S.

23. The peptide or the pharmaceutically acceptable salt, solvate, or prodrug thereof according to any one of claims 1-22, wherein the peptide is chemically modified, e.g., PEG-modified, lipid-modified, D-type amino acid replaced, or non-naturally occurring amino acid replaced.

24. A peptide or a pharmaceutically acceptable salt, solvate or prodrug thereof, wherein the peptide is selected from any one of SEQ ID NOs: 1 to 49, or differs therefrom by the substitution, deletion or addition of 1 or 2 amino acids in the amino acid sequence, preferably the substitution, deletion and addition occur at amino acid residues other than those of the core motif (DXED), e.g., being conservative amino acid substitution.

25. A peptide selected from any one of SEQ ID NOs: 6, 22, 25, 26, 33, 40, 43, 44, and 46 or a pharmaceutically acceptable salt, solvate or prodrug thereof.

26. A method for preparing the peptide or the pharmaceutically acceptable salt, solvate or prodrug thereof according to any one of claims 1-25, preferably the method comprises synthesizing the peptide using a solid-phase synthesis.

27. A pharmaceutical composition comprising the peptide or the pharmaceutically acceptable salt, solvate or prodrug thereof according to any one of claims 1-25, and a pharmaceutically acceptable carrier.

28. A method for preventing or treating a TGF-β related disease, comprising administering to a subject in need thereof a prophylactically or therapeutically effective amount of the peptide or the pharmaceutically acceptable salt, solvate or prodrug thereof according to any one of claims 1-25, or the pharmaceutical composition according to claim 27;
optionally, the method further comprises administering to the subject an effective amount of a second therapeutic agent;
preferably, the disease has macrophage infiltrations at the site of the lesion, or is associated with tissue damage, inflammation or fibrosis.

29. The method according to claim 28, wherein the TGF-β related disease is a fibrotic disorder, preferably selected from: pulmonary fibrosis (e.g., IPF), hepatic fibrosis, renal fibrosis, myelofibrosis, myocardial fibrosis, and/or dermal fibrosis.

30. The method according to claim 28, wherein the TGF-β related disease is a solid tumor, preferably selected from: lung cancer, liver cancer, breast cancer, uterine cancer, prostate cancer, pancreatic cancer, colon cancer, skin cancer, central nervous system cancer, fibromyoma, fibroma, fibroadenoma, and fibrosarcoma.

31. Use of the peptide or the pharmaceutically acceptable salt, solvate or prodrug thereof according to any one of claims 1-25, for *in vitro* or *in vivo:*
- blocking the binding of CD36 to TSP-1;
- inhibiting TSP-1-dependent TGF-β1 activation;
- decreasing the amount of active TGF-β1 in fibrotic tissue;
- inhibiting TGF-β1-mediated downstream signaling;
- reducing collagen deposition in fibrotic tissue;
- inhibiting inflammatory and/or fibrotic lesions mediated by TGF-β1;
- inhibiting extracellular matrix-related gene expressions stimulated by TGF-β1;
- inhibiting tumor cell migration;
- preventing or treating TGF-β1 related diseases, especially fibrosis or cancer; or in the manufacture of a medicament for the above uses.
